# EUROPEAN PATENT APPLICATION

(11) **EP 4 506 464 A1**
(43) Date of publication of application: **12.02.2025**
(21) Application number: 23189907.1
(22) Date of filing: 07.08.2023
(51) Int. Cl.: C12Q 1/6823

(54) **CRISPR-COACERVATE SYSTEM**

(71) Applicant: ETH Zurich, 8092 Zurich (CH)
(72) Inventor: DEMELLO, Andrew, 8044 Zürich (CH); AROSIO, Paolo, 8046 Zürich (CH); CAPASSO PALMIERO, Umberto, 8049 Zürich (CH); PAGANINI, Carolina, 8005 Zürich (CH); RICHARDS, Daniel, 8165 Schöfflisdorf (CH); LESINSKI, Jacob, 8037 Zürich (CH)

(57) **Abstract**

The present invention pertains to a composition or kit of parts comprising (1) coacervate-forming compounds, (2) a Cas complex comprising a guide RNA and a Cas protein, wherein the Cas protein has trans or collateral cleavage activity for a nucleic acid sequence of a reporter, (3) a reporter comprising a detection moiety covalently linked to the nucleic acid sequence, wherein the nucleic acid sequence is suitable for being cleaved by the Cas complex, and the detection moiety is suitable for optical or electrochemical detection; and (4) optionally a target suitable for activating the Cas complex, wherein at least one, two or three of the coacervate-forming compounds, the Cas complex, the reporter, the cleaved detection moiety and/or the target are configured such that at least one, two or three of the Cas complex, the reporter, the cleaved detection moiety and/or the target are taken up by coacervates formed by the coacervate-forming compounds. The present invention further pertains to associated uses and methods for detection.

## Description

The present invention pertains to a composition or kit of parts comprising coacervate-forming compounds, a Cas complex comprising a guide RNA and a Cas protein, a reporter comprising a detection moiety covalently linked to the nucleic acid sequence; and optionally a target suitable for activating the Cas complex. The present invention further pertains to associated uses and methods for detection.

CRISPR is a powerful diagnostic tool with high specificity and sensitivity, and rapid adaptability to different targets. However, the enzyme kinetics that drive CRISPR-based diagnostics are slow necessitating lengthy diagnostic tests and/or a nucleic acid amplification step to increase the speed and decrease the detection limit of the assay. This amplification step increases the cost of the reagents for the assay, the time and complexity of the assay, as well as the complexity of microfluidic chips and complementary devices used to run detection assays based on the CRISPR technology. These limitations hamper the use of the CRISPR technology for affordable and rapid point-of-care molecular diagnostics.

It is the objective underlying the present invention to provide improved, in particular simple, fast and efficient detection tools, detection systems and corresponding detection methods based on CRISPR technology.

The above problem is solved by the composition or kit of parts according to the appended claims.

In a first aspect, the present invention is directed to a composition or kit of parts comprising:
(1) coacervate-forming compounds,
(2) a Cas complex comprising a guide RNA and a Cas protein, wherein the Cas protein has trans or collateral cleavage activity for a nucleic acid sequence of a reporter,
(3) a reporter comprising a detection moiety covalently linked to the nucleic acid sequence, wherein the nucleic acid sequence is suitable for being cleaved by the Cas complex, and the detection moiety is suitable for optical detection, optionally fluorescence, colorimetric, or luminescence detection, or electrochemical detection; and
(4) optionally a target suitable for activating the Cas complex,
wherein
at least one, optionally at least two or three, of the coacervate-forming compounds, the Cas complex, the reporter, the cleaved detection moiety and/or the target are configured such that at least one, optionally at least two or three, of the Cas complex, the reporter, the cleaved detection moiety and/or the target are taken up by coacervates formed by the coacervate-forming compounds.

All definitions, examples and explanations provided herein are valid for all aspects and embodiments of the present invention if not specifically indicated otherwise or in contradiction.

Coacervate-forming compounds, as used herein, are known in the art (see, e.g., Wu et al. Supramolecular Materials 1 (2022) 100019) and form a liquid-liquid phase separation. A coacervate is an aqueous phase rich in (macro)molecules such as, e.g., synthetic polymers, proteins or nucleic acids. It forms through liquid-liquid phase separation (LLPS), leading to a dense phase in thermodynamic equilibrium with a dilute phase. Coacervates or coacervation as used herein includes coacervation based on segregative phase separation and associative phase separation. Segregative phase separation refers to the formation of aqueous two-phase systems, in which two or more soluble macromolecules (e.g. polymers) or a macromolecule with a small molecule (e.g. phosphate, sulfate or citrate) form two immiscible phases above a threshold concentration in equilibrium. Associative phase separation refers to phase separation of a single solute component, i.e. simple coacervation, or of two or more solute components, i.e. complex coacervation, that condense into one concentrated liquid phase in equilibrium with a dilute phase.

In some examples, coacervates can be liquid droplets, for example with an essentially spherical shape, e.g. of about 0.2 to 100 µm in diameter, that are able to coalesce, e.g. to form larger droplets or a continuous phase. Moreover, and for example, the droplets can, e.g., exhibit recovery in fluorescence recovery after photobleaching (FRAP) experiments, sometimes within seconds. Coacervate-forming compounds, as used herein, can be any compounds that form coacervates in an aqueous phase, e.g. (synthetic) polymers, proteins or nucleic acids. The terms "coacervates" and "(liquid) droplets" are used interchangeably herein and also include aqueous two-phase systems.

The droplets in the context of the present invention can be multiphase immiscible droplets, wherein compartments of at least two different droplets can be fully immiscible or form subcompartments.

In general, reference is made to WO2022/122675, incorporated by reference in its entirety, which discloses suitable coacervate-forming compounds and their synthesis within the context of the present invention.

The term "Cas complex" as used herein is also commonly known as "CRISPR-Cas complex" or "ribonucleoprotein (RNP)" and these terms are used interchangeably. The Cas complex for use in the present invention can be any Cas complex having activity within the context of the CRISPR technology and having trans or collateral cleavage activity or inducing a cascade of reactions which have the same result as trans cleavage activity (i.e. a series of chemical reactions that lead to endonuclease activity (of a separate enzyme) after the cis cleavage of a Cas protein - CRISPR associated proteins). The nucleic acid of the reporter is chosen such that it is cleaved by the Cas protein of the Cas complex of the composition or kit of parts described herein. Cleavage means that at least one covalent bond between two nucleic acids or to a nucleic acid is cleaved. The nucleic acid sequence mentioned above is linked, e.g. covalently bound or associated by ionic interactions, to the detection moiety of the reporter and upon cleavage by the Cas protein, the nucleic acid sequence remaining attached to the detection moiety is either shortened by at least one nucleic acid, or removed entirely from the detection moiety, resulting in the "cleaved detection moiety" as used herein. The shortening or removal of this nucleic acid sequence changes the properties, e.g. the steric properties, the electronic/electrostatic properties, and/or the hydrophilicity of the detection moiety. The reporter may comprise or consist of the detection moiety and the nucleic acid sequence.

The detection moiety can be any organic or inorganic moiety that can be detected by or that induces a secondary reaction detectable by optical (e.g. fluorescence, colorimetric, or luminescence detection) or electrochemical methods known in the art.

The optional target is the target which activates the cleavage activity of the Cas complex towards the nucleic acid sequence of the reporter.

Upon combination of the coacervate-forming compounds, at least one of the Cas-complex, the reporter, the optional target, and the cleaved detection moiety are taken up by coacervates formed by the coacervate-forming compounds. However, the Cas complex or protein may optionally also be (covalently) bound to the coacervate-forming compounds or to a TBM (e.g. charged polymers) such that the Cas complex or protein is allocated to the coacervates and displayed towards the inside or outside of the coacervates formed by the coacervate-forming compounds. Alternatively or additionally, the reporter or at least the nucleic acid sequence linked to the detection moiety can be (e.g. covalently) bound to the coacervate-forming compounds or to a TBM (e.g. for hydrophobic molecules) in analogy to the example given for Cas above.

Depending on the choice of the coacervate-forming compounds, the reporter, the nucleic acid sequence, and optionally the target, the interactions between these components and the uptake of the components into the coacervates can be controlled, e.g. based on electrostatic interactions or specific target-binding moieties attached to one or more of the components. This choice and control are within the purview of the skilled person and can be verified by standard, e.g. optical, tests such as microscopy. For example, reaction speeds with and without coacervates can be compared to determine whether a component enters the coacervates or not. The uptake of the components or the display thereof in or on the coacervates formed by the coacervate-forming compounds leads to a local increase in the components' concentration and/or brings the components into closer proximity with each other, and, thus, causes an acceleration of the cleavage reaction of Cas and/or an increase in local optical or electrochemical readout, which in turn obviates or reduces the need for time-consuming amplification of the target for the Cas complex.

For example, uptake can be warranted by the negative charge on nucleic acids in the context of the reporter and/or the target, which negative charge will cause the nucleic acids to be taken up into the coacervates, e.g. if the coacervate-forming compounds are chosen such that the coacervates are essentially or at least partially positively charged (e.g. also depending on the pH or on the ionic strength of the environment). The exemplary co-localization of the Cas complex and the optional target can accelerate the reaction between the Cas complex and the target sufficiently so that the reporter could be located outside the coacervates and still giving a sufficiently high readout without the need for (target) nucleic acid amplification. The co-localization of the Cas complex and the nucleic acids (or any other component of the composition described herein) can be achieved based on interaction(s) between the components or intermolecular forces between the components and the liquid phase (interactions include electrostatic interactions, hydrogen bonding, van der Waals interactions or hydrophobic interactions), or by (covalently) attaching the Cas complex and/or the reporter to the coacervate-forming compounds or a TBM.

After the uptake of, e.g. the Cas complex and the target, from outside the droplets into the droplets, the nucleic acid attached to the detection moiety of the reporter can be cleaved (see above, also referred to as the "cleaved detection moiety" herein) by the Cas protein whereupon the cleaved detection moiety may either remain within the coacervate, be expelled/released therefrom or be taken up in separate coacervates, depending on the choice of nucleic acid-detection moiety and/or coacervate-forming compounds and the altered chemical composition or properties thereof. In this example, the reporter may be taken up by the coacervate ("enter the coacervate"), but it does not need to be concentrated compared to the aqueous phase.

The uptake into the coacervates may also be controlled by external stimuli, e.g. adjusting the environment of the liquid droplets, e.g. by adjusting the pH of the aqueous medium, the temperature, or the ionic strength of the aqueous medium. Also, the addition of further compounds to the environment of the liquid droplets (the aqueous medium) such as salts, proteins, antibodies or vesicles can change the environment and lead to an external stimulus that controls the uptake of different components of the composition or kit of parts described herein. The controlled uptake does not necessarily correlate with the formation or dissolution of the liquid droplets. For example, a component can be taken up by the liquid droplets at a given ionic strength of the aqueous medium and be released at a different, e.g. higher ionic strength, without dissolving the liquid droplets.

The process described herein for uptake of compounds from outside the droplets into the droplets is not to be confused with the loading of, e.g. solid, vesicles in the context of drug delivery. The uptake refers to a relative increase of concentration while the components can enter and exit the coacervates but wherein the kinetics of entry and exit depends on the chemical composition of the components and/or the coacervates.

Further exemplary details on the uptake and the possible interactions between the components of the present composition or kit of parts are given further below.

Exemplary Cas proteins and nucleases include
- cas12a, for example LbCas12a, AsCas12a, and FnCas12a (J. Chen et al., Science (2018), 360, 6387, 436-439).
- cas12b, for example AacCas12b, AapCas12b, and BrCas12b (L.T. Nguyen et al., eBioMedicine (2022), 77, 103926).
- cas13, for example LbaCas13a, PsmCas13b, LbuCas13a, and CCaCas13b (J.S. Gootenberg et al., Science (2018), 360, 6387, 439-444).
- cas14, for example cas14a1, cas14b1, and cas14u1 (L.B. Harrington et al., Science (2018), 362, 6416, 839-842).
- cas7, for example Cmr1, Cmr4, and Cmr6 (J.A. Steens et al., Nature Communications (2021), 21, 5033)
- cas10, for example Cmr2 (J.A. Steens et al., Nature Communications (2021), 21, 5033)
- cas5, for example Cmr3 (J.A. Steens et al., Nature Communications (2021), 21, 5033)
- cas11, for example Cmr5 (J.A. Steens et al., Nature Communications (2021), 21, 5033)
- TTHB144 (J.A. Steens et al., Nature Communications (2021), 21, 5033) Exemplary nucleic acid sequences for guide-RNAs include
- UAAUUUCUACUAAGUGUAGAUugaaguagauauggcagcac (SEQ ID NO: 1), UAAUUUCUACUAAGUGUAGAUacaauaugugcuucuacaca (SEQ ID NO: 2) for Cas12, specifically LbCas12a (J. Chen et al., Science (2018), 360, 6387, 436-439).
- GAAGGUGGUUAGCUACAGGCUGACCAGUGCAGUUGUGUCAUGUGCUACGGUGACCUAACACGUCA CUCAGUCACAACGGCUAUCUAUAUUUCCACUAACCAAAGUUAGUGGAAAUGUAGAUGGUUAGCACC GAAGAACGCUGAAGCGCUG (SEQ ID NO: 3) for Cas12, specifically BrCas12b (L.T. Nguyen et al., eBioMedicine (2022), 77, 103926).
- GACCACCCCAAAAATGAAG GGGACTAAAACATGCTTCT GTCCAGTGAGCATGG (SEQ ID NO: 4) for Cas13, specifically LbuCas13a
- TGACTCCCTAGAACCACGACAGTTTGCCTTGTTGTAGA AGCTTATCGTTTGGATAGGTATGACAAC (SEQ ID NO: 5) for Cas13, specifically PsmCas13a
- GTTGATGAGAAGAGCCCAAGATAGAGGGCAATAACACT CCCTAGAACCACGACAGTTTGCCTT (SEQ ID NO: 6) for Cas13, specifically LbaCas13a (J.S. Gootenberg et al., Science (2018), 360, 6387, 439-444).
- TTCACTGATAAAGTGGAGAACCGCTTCACCAAAAGCTGTCCCTTAGGGGATTAGAACTTGAGTGAAGGTG GGCTGCTTGCATCAGCCTAATGTCGAGAAGTGCTTTCTTCGGAAAGTAACCCTCGAAACAAATTCATTTga aaGAATGAAGGAATGCAACacttgacacttaatgctcaa (SEQ ID NO: 7) for Cas14, specifically Cas14a1 (L.B. Harrington et al., Science (2018), 362, 6416, 839-842).
- CUUCACUGAUAAAGUGGAGAACCGCUUCACCAAAAGCUGUCCCUUAGGGGAUUAGAACUUGAGUGA AGGUGGGCUGCUUGCAUCAGCCUAAUGUCGAGAAGUGCUUUCUUCGGAAAGUAACCCUCGAAACAA AUUCAUUUGGAAUGCAACGAAUGAAGGAAUGCAACUCAACUAAUGUAACU (SEQ ID NO: 8) for Cas14, specifically Cas14a1 (J. Hu et al., Chemical Communications (2021), 57, 10423-10426).
- AUUGCGACCACACAAUCGAAGCGCAGUAAGGAUGGCUAGUGUAACU (SEQ ID NO: 9) for Crm#, such as Cas5, Cas7, Cas10, Cas11 (J.A. Steens et al., Nature Communications (2021), 21, 5033). Exemplary reporters for fluorescent readout and nucleic acid sequences to be cleaved by Cas include
- forCas12:
   FAM-CCCCCC-BHQ1 (SEQ ID NO: 10), FAM-AAAAAA-BHQ1 (SEQ ID NO: 11), FAM-TTTTTT-BHQ1 (SEQ ID NO: 12), FAM-GGGGGG-BHQ1 (SEQ ID NO: 13) (H. Yue et al., Nano Letters (2021), 21, 4643-4653),
   HEX-TTTTTTTT-IABkFQ (SEQ ID NO: 14), cy5-TTTTTTT-BHQ2 (SEQ ID NO: 15) (L.T. Nguyen et al., eBioMedicine (2022), 77, 103926)
   FAM-TTATT- IABkFQ (SEQ ID NO: 16), FAM-rUrUrArUrU- IABkFQ (SEQ ID NO: 17) (J. Chen et al., Science (2018), 360, 6387, 436-439)
- for Cas14 :
   FAM-TTTTT-BHQ1 (SEQ ID NO: 18), FAM-TTTTTT-BHQ1 (SEQ ID NO: 19), FAM-TTTTTTT-BHQ1 (SEQ ID NO: 20), FAM-TTTTTTTT-BHQ1 (SEQ ID NO: 21), FAM-TTTTTTTTT-BHQ1 (SEQ ID NO: 22), FAM-TTTTTTTTTT-BHQ1 (SEQ ID NO: 23), FAM-TTTTTTTTTTT-BHQ1 (SEQ ID NO: 24), FAM-TTTTTTTTTTTT-BHQ1 (SEQ ID NO: 25) (L.B. Harrington et al., Science (2018), 362, 6416, 839-842) FAM-TTTTTTTTTTTTTTT-BHQ1 (SEQ ID NO: 26) (J. Hu et al., Chemical Communications (2021), 57, 10423-10426)
- for Cas13 : FAM-rUrUrUrUrU- IABkFQ (SEQ ID NO: 27), HEX-rUrUrUrUrU- IABkFQ (SEQ ID NO: 28), cy5-rUrUrUrUrU- IAbRQSp (SEQ ID NO: 29), FAM-rArArArArA- IABkFQ (SEQ ID NO: 30), FAM-mArArUrGrGrCmAmArArArUrGrGrCmA-Biotin (SEQ ID NO: 31), FAM-rCrCrCrCrC- IABkFQ (SEQ ID NO: 32), FAM-rGrGrGrGrG- IABkFQ (SEQ ID NO: 33), FAM-TrCrGrArArUrG- IABkFQ (SEQ ID NO: 34) (J.S. Gootenberg et al., Science (2018), 360, 6387, 439-444).
- for Crm# such as Cas5, Cas7, Cas10, Cas11 :
   FAM-TAGTTGTGATGCWATCATGACTAG-TAMRA (SEQ ID NO: 35) (J.A. Steens et al., Nature Communications (2021), 21, 5033)
- for auxiliary proteins such as TTHB144 :
   cy5-AAACGACGGCCAGUGCCAAGCUUACUAUACAACCUACUACCUCAU-quencher (SEQ ID NO: 36) (J.A. Steens et al., Nature Communications (2021), 21, 5033)

Exemplary reporters for electrochemical readout and nucleic acid sequences to be cleaved by Cas include:
- for Cas12a
   (MethyleneBlue-Gold Nano Particles - Streptavidin) - SH-ATGTTGTTCTGCCCATCG-Biotin (SH = Thiol) (SEQ ID NO: 37) (Y. Lee et al., Sensors and Actuators B: Chemical (2021), 326, 128677) -SH-TTTTAATTTT-Methylene Blue (SEQ ID NO: 38) (Y. Dai et al., Angewandte Chemie (2019), 131, 48, 17560-17566)
- for Cas13a
   -SH-C6spacer- rUrUrUrUrUrUrUrUrUrUrUrUrUrUrUrUrUrUrUrU-Methylene Blue (SEQ ID NO: 39) (J. Yang et al., Nature Chemical Biology (2023), 19, 45-54).

Suitable targets which are suitable for activating the Cas complex are known in the art and may be DNA or RNA sequences.

In general, it is known in the art how to design a Cas complex and nucleic acid sequence to be cleaved by the Cas protein for a target of interest. Reference is made to J. Chen et al., Science (2018), 360, 6387, 436-439; J.S. Gootenberg et al., Science (2018), 360, 6387, 439-444; L.B. Harrington et al., Science (2018), 362, 6416, 839-842.

In a general example, the reporters are typically optimized and are kept the same for a specific protein. The Cas protein is, e.g., selected according to what needs to be cut (some cut RNA, some dsDNA, some ssDNA). Each protein has a corresponding guide-RNA that specifically binds to it through an RNA segment called the scaffold or repeat. The scaffold or repeat does not change for the corresponding protein. The part that changes and is specific to the target nucleic acid is the spacer. The spacer is complementary to the target nucleic acid of interest and is bound to the repeat.

For example, the present composition or kit of parts may be one,
wherein the coacervate-forming compounds, the Cas complex, the reporter, the cleaved detection moiety and the target are configured such that at least the Cas complex, the reporter, the cleaved detection moiety and the target are taken up by coacervates formed by the coacervate-forming compounds; or
wherein at least the coacervate-forming compounds and the Cas complex are configured such that at least the Cas complex, is taken up by coacervates formed by the coacervate-forming compounds; or
wherein at least the coacervate-forming compounds, the Cas complex, and the reporter are configured such that at least the Cas complex and the reporter are taken up by coacervates formed by the coacervate-forming compounds; or
wherein at least the coacervate-forming compounds, the Cas complex, the reporter and the cleaved detection moiety are configured such that at least the Cas complex, the reporter, and the cleaved detection moiety are taken up by coacervates formed by the coacervate-forming compounds; or
wherein at least the coacervate-forming compounds, the Cas complex and the target are configured such that at least the Cas complex and the target are taken up by coacervates formed by the coacervate-forming compounds; or
wherein at least the coacervate-forming compounds, the Cas complex, the reporter, the cleaved detection moiety and the target are configured such that at least the Cas complex, the reporter, the cleaved detection moiety and the target are taken up by coacervates formed by the coacervate-forming compounds; or
wherein at least the coacervate-forming compounds, the Cas complex, the reporter, and the target are configured such that at least the Cas complex, the cleaved detection moiety, and the target are taken up by coacervates formed by the coacervate-forming compounds; or
wherein at least the coacervate-forming compounds, the reporter and the cleaved detection moiety are configured such that at least the cleaved detection moiety is taken up by coacervates formed by the coacervate-forming compounds; or
wherein at least the coacervate-forming compounds and the reporter are configured such that at least the reporter and the cleaved detection moiety is taken up by coacervates formed by the coacervate-forming compounds; or
wherein at least the coacervate-forming compounds are configured such that at least the target is taken up by coacervates formed by the coacervate-forming compounds.

In all the above examples, the expression «wherein the Cas complex is configured such that the Cas complex is taken up by coacervates formed by coacervate-forming compounds» is to be understood that it includes the option that the Cas complex is bound to (e.g. by covalent bonds and/or by co-expression) at least part of the coacervates as described herein, to a coacervate-forming compound as described herein or to a TBM that interacts with the coacervates (e.g. by electrostatic interactions) as defined herein.

In an embodiment of the composition or kit of parts according to the present invention which may be combined with any of the embodiments of the composition or kit of parts preaddressed or still to be addressed unless in contradiction, the composition or kit of parts according to the present invention is one, wherein
(A) the detection moiety is selected from the group consisting of: one or more fluorophore(s) covalently bound via the nucleic acid to one or more quenchers; metal nanoparticles, optionally gold nanoparticles; a molecule suitable for colorimetric analysis, optionally horseradish peroxidase, calf intestine alkaline phosphatase, 3,3',5,5'-tetramethylbenzidine (TMB), 2,2'-azinobis [3-ethylbenzothiazoline-6-sulfonic acid]-diammonium salt (ABTS), *o-*phenylenediamine dihydrochloride (OPD), *p*-nitrophenyl Phosphate (*p*-NPP), luminol, polyphenols, acridine esters, and luciferin; and a redox-active moiety suitable for electrochemical detection;
(B) the detection moiety is one or more fluorophore(s) covalently linked to the nucleic acid sequence and one or more quencher(s) for the fluorophore(s), wherein the nucleic acid is suitable for being cleaved by the Cas complex to separate the fluorophore(s) and the quencher(s); and/or
(C) the nucleic acid sequence is a ssDNA, ssRNA, dsDNA, dsRNA, DNA hairpin, a locked nucleic acid (LNA) or peptide nucleic acid (PNA), wherein the nucleic acid optionally comprises or consists of artificial nucleic acid base pair analogs.

If the detection moiety is comprised of a fluorophore covalently bound via the nucleic acid to a quencher, the quencher and/or the fluorophore can be constructed such that either of them, or both, are retained in the coacervates after cleavage or that e.g. the quencher or the fluorophore is expelled from the coacervates after cleavage. Accumulation of the fluorophore inside the coacervates and in the absence of the quencher will, e.g. increase the signal strength for fluorescence readout. Also, other detection moieties (e.g. as mentioned herein, e.g. metal nanoparticles, molecules for colorimetric assays or redox-active moieties) can, in some embodiments, be bound to a compound that interacts with the coacervates (e.g. a charged polymer, a phase separating unit or a hydrophobic polymer).

In an embodiment of the composition or kit of parts according to the present invention which may be combined with any of the embodiments of the composition or kit of parts preaddressed or still to be addressed unless in contradiction, the composition or kit of parts according to the present invention is one, wherein the coacervate-forming compounds comprise or consist of
a. a self-associating polymer,
b. cationic and anionic molecules, optionally mixed in ratios so that a coacervate including at least the Cas complex, the target, the reporter or the cleaved detection moiety is formed, optionally in ratios between 0.2 mol/mol to 0.8 mol/mol cationic to anionic, optionally polycations, polyanions, polyU, proteins, peptides, nucleic acids, small molecules, optionally ATP, ADP, counterions, poly-lysine, poly-arginine, poly-aspargine, polyethylenimine, polyamidoamine, mannosylated polyethylenimine, or chitosan, and
c. polyethylene glycol, dextran, sucrose, polysucrose, glycerol, sorbitol, copolymerized sucrose and epichlorohydrin, Ficoll, poly(2-ethyl-2-oxazoline), poly(vinyl alcohol), glucose, bovine serum albumin, ionic liquids, double hydrophilic block copolymers, optionally pullulan-b-poly(N,N-dimethylacrylamide), polyethylene glycol)-b-poly(2-methyl-2-oxazoline), poly(ethylene glycol)-b-dextran, poly(ethylene glycol)-b-pullulan, dextran-b-poly(sarcosine)), or a combination of these, optionally a combination with alcohols, salts and/or amino acids, optionally ethanol, 1-propanol, 2-propanol, phosphates, sulfates or citrates.

Examples for cationic and anionic molecules for use in the present invention include polyethylenimine, polyamidoamine, polyacrylates and polymethacrylates (e.g. poly[2 (dimethylamino)ethyl methacrylate], poly(2-dimethylamino)ethyl methacrylate) methyl chloride), polypeptides (e.g. poly-lysine, poly-arginine, poly-aspargine), allyl polymers (e.g. poly(allylamine hydrochloride, poly(diallyldimethylammonium chloride), polyesters, polysaccharides (e.g. Q-amylose, mannosylated polyethylenimine, chitosan); poly(methyl vinyl ether-alt-maleic acid), polyacrylates and polymethacrylates (e.g. polyacrylic acid, poly(methacrylic acid), polystyrene sulfonate, poly(3-Sulfopropyl methacrylate)), polysaccharides (e.g. Cm-amylose), polypeptides (e.g. poly(glutamic acid)); proteins, peptides, nucleic acids, optionally polyU, or small molecules, optionally ATP, ADP, counterions, spermine, spermidine. The cationic and anionic molecules can, e.g., include the cationic and anionic components of the assay, i.e. the target DNA can serve as the anionic molecule, and a Cas protein bound to a positively-charged or negatively-charged macromolecule can serve as the cationic or the anionic molecule, respectively.

As used herein, peptides comprise between 2 and 50 amino acids and polypeptides, optionally proteins, include more than 50 amino acids.

In an embodiment of the composition or kit of parts according to the present invention which may be combined with any of the embodiments of the composition or kit of parts preaddressed or still to be addressed unless in contradiction, the composition or kit of parts according to the present invention is one, wherein the self-associating polymer is selected from the group consisting of:
(A) a polymer exhibiting phase transition when the temperature is decreased below the critical solution temperature of the polymer, optionally a zwitterionic polymer, ureido polymers and optionally derivatives thereof, optionally poly(allylamine)-co-poly(allylurea), poly(allylurea), poly(ureidoethylmethacrylate), poly(citrulline), poly(allylamine)-co-poly(allylurea) and optionally derivatives thereof, poly(N-acryloylglycinamide) and optionally derivatives thereof, poly(*N*-acryloylasparaginamide) and optionally derivatives thereof, poly(*N*-(2-hydroxypropyl) methacrylamide)-glycolamide) and optionally derivatives thereof, or copolymers of acrylamide and acrylonitrile,
(B) a polymer exhibiting phase transition when the temperature is increased beyond the critical solution temperature of the polymer, optionally poly(N-isopropyl acrylamide) (PNIPAM), poly(2-(N-(dimethylamino) ethyl methacrylate) (PDMAEMA), polyethylene glycol) methacrylate (PEGMA), poly(N-vinyl caprolactam) (PNVCL), poly(phenylene oxide) (PPO), poly(2-oxazolines) (Pox), poly(N,N-diethyl acrylamide), poly(oligoethylene glycol [methyl ether] [meth]acrylates), poly(methyl vinyl ether), polypropylene glycol), and
(C) a protein or peptide exhibiting phase transition, optionally
   a. an elastin-like polypeptide comprising or consisting of at least two repeats having the amino acid formula (V-P-G-X-G)ₙ (SEQ ID NO: 40), wherein X is any natural amino acid except proline and n is an integer from 2 to 600, or
   b. a low complexity domain (LCD) stretch of naturally occurring nucleic acid binding proteins,
      - optionally of transcription factors or RNA binding proteins, optionally selected from the group consisting of Dhhl, Dbpl, Lafl, Ddx4, hNRNPA1, FUS, EWS and hnRNPNA2,
      - optionally proteins comprising or consisting of an amino acid sequence having at least 85%, 87.5%, 90%, 92%, 94%, 95%, 96%, 97%, 98%, or 99% sequence identity and/or amino-acid composition identity to Dhhl, Dbpl, Lafl, Ddx4, hNRNPA1, FUS, EWS or hnRNPNA2, optionally over the whole sequence, and
      - proteins in which not more than 5, optionally not more than 3, 2 or 1 amino acid(s) is (are) removed from the sequence of the RNA binding proteins selected from the group consisting of Dhhl, Dbpl, Lafl, Ddx4, hNRNPA1, FUS, EWS and hnRNPNA2 at one or both ends thereof,
   c. a resilin-like polypeptide comprising or consisting of at least two identical or different repeats having the amino acid formula (GRGDSPYS)ₙ (SEQ ID NO: 41) or (GGRPSDSYGAPGGGN)ₙ (SEQ ID NO: 42) or (GYSGGRPGGQDLG)ₙ (SEQ ID NO: 43)or (AQTPSSQYGAP)ₙ (SEQ ID NO: 44), wherein n is an integer from 2 to 600, optionally a resilin-like polypeptide comprising or consisting of an amino acid sequence having at least 50%, optionally at least 75% or 90%, sequence identity and/or amino-acid composition identity to the resilin-like polypeptide comprising or consisting of at least two identical or different repeats having the amino acid formula (GRGDSPYS)ₙ (SEQ ID NO: 41) or (GGRPSDSYGAPGGGN)ₙ (SEQ ID NO: 42) or (GYSGGRPGGQDLG)ₙ (SEQ ID NO: 43) or (AQTPSSQYGAP)ₙ (SEQ ID NO: 44), wherein n is an integer from 2 to 600.

The low complexity domain (LCD) stretch is also known as intrinsically disordered region (IDR). LCDs for use in the present invention are known in the art and include those, e.g., exemplified in WO2020048996A1, specifically in claim 4, incorporated by reference in its entirety.

The zwitterionic polymer, as used herein, may have an overall, partial or localized (e.g. inside vs. outside the coacervates) positive, negative or neutral charge.

A protein or peptide exhibiting phase transition, as used herein, is a protein that spontaneously separates into a macromolecule-rich and a macromolecule-depleted phase in response to stimuli such as ionic strength, temperature, or pH in an aqueous phase. These proteins or peptides (as well as the zwitterionic polymers) can be fused (e.g. covalently bound or co-expressed) to Cas of the Cas complex as described herein.

In an embodiment of the composition or kit of parts according to the present invention which may be combined with any of the embodiments of the composition or kit of parts preaddressed or still to be addressed unless in contradiction, the composition or kit of parts according to the present invention is one, wherein the self-associating polymer, optionally zwitterionic polymer, is according to Formula (I): 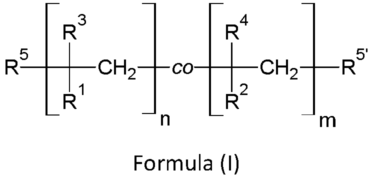 wherein
**n** is an integer selected from 0 to 2500, optionally from 25 to 250;
**m** is an integer selected from 2 to 2500, optionally from 25 to 250;
**R³** and **R⁴** are each independently selected from the group consisting of hydrogen and methyl;
**R⁵** and **R^{5'}** are independently selected from
   - hydrogen, linear or branched (C₁₋₁₀)alkyl, a halide and phenyl, or
   - a group resulting from a polymerization initiator, optionally a group resulting from a RAFT polymerization initiator, optionally 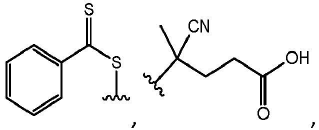 or a target binding moiety (TBM), optionally a TBM attached to or reacted with a group resulting from a polymerization initiator, optionally from a RAFT polymerization initiator, optionally 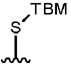 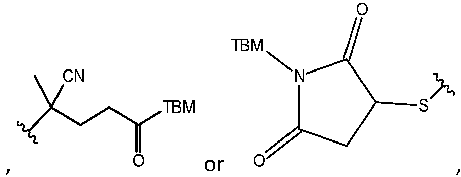 wherein the TBM is optionally selected from the group consisting of azide, alkyne, carboxylate, amine, thiol, hydroxyl, aldehyde, cyanate, sulfonyl, tosyl, tresyl, epoxide, carbonate, anhydride, carbamate, imidazole, azlactone, triazine, maleimide, aziridine, peroxide, acyl, anthraquinone, diazo, diazirine, psoralen, NHS ester, imido ester, (C₂₋₁₀)alkenyl or alkyl groups, DBCO, cytosine, guanidine, streptavidin, avidin, biotin, an aptamer, a peptide, optionally a peptide comprising an affinity tag, optionally an affinity tag selected from FLAG, HIS and ALFA, a protein, an oligonucleotide, a positively or negatively charged oligopolymer, a molecularly imprinted polymer, an affinity polymer, a nucleic acid, a carbohydrate, a dye, an antibody, an organic and inorganic nanoparticle, and chelating agents, optionally ethylenediaminetetraacetic acid (EDTA);
**R¹** is selected from the group consisting of
   (i) -X-hydrogen, -X-methyl, -X-ethyl and -X-propyl;
   (ii) 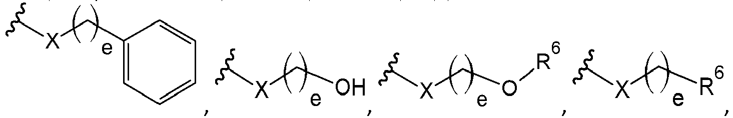 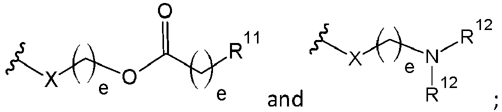
   (iii) 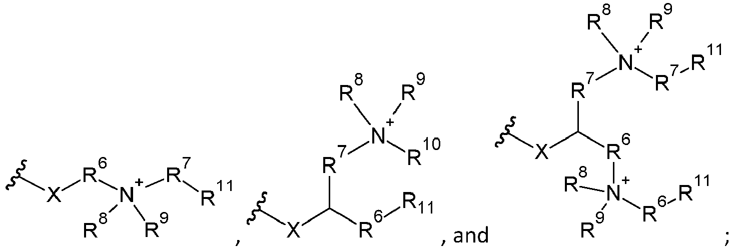
   (iv) 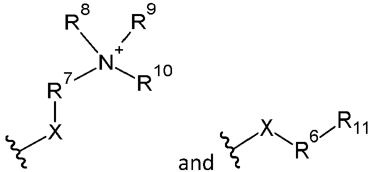 in a ratio of about 1:1 within the polymer;
   (v) 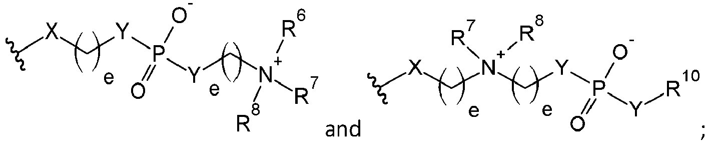 and
   (vi) 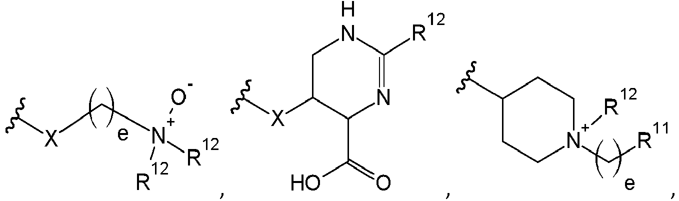 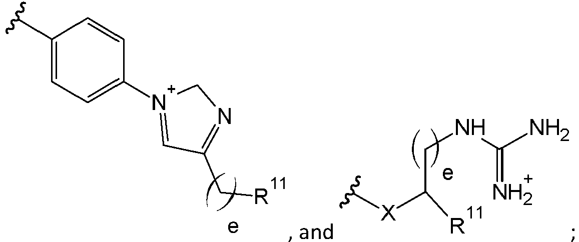
R² is selected from the group consisting of 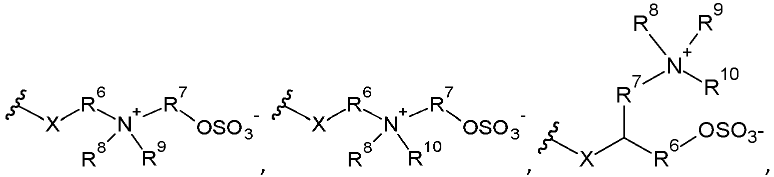 and 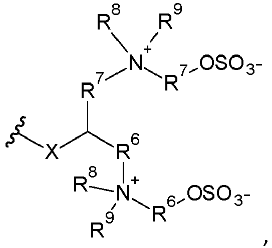 wherein
   **X** is selected from the group consisting of -C(=O)-O-, -C(=O)-NH-, aliphatic and aromatic carbo- or heterocycle;
   **Y** is oxygen or absent;
   **e** is an integer selected from 1 to 5, optionally from 2 to 3;
   **R⁶** and **R⁷** are each independently selected from the group consisting of
      linear or branched, substituted or non-substituted (C₁₋₁₀)alkyl, optionally (C₁₋₅)alkyl, optionally methyl, ethyl and propyl,
      linear or branched, substituted or non-substituted (C₁₋₁₀)alkyl, optionally (C₂₋₆)alkyl chains, comprising one or more heteroatoms selected from the group consisting of O, N, S and P, optionally (C₂₋₅)alkyl ethers,
      (C₂₋₁₀)alkenyl,
      phenyl, and an aromatic heterocycle, optionally pyridine, pyrrole, furan and imidazole;
   **R⁸, R⁹** and **R¹⁰** are each independently selected from the group consisting of hydrogen, linear or branched, substituted or non-substituted (C₁₋₁₀)alkyl, optionally (C₁₋₅)alkyl, optionally methyl, ethyl and propyl, (C₂₋₁₀)alkenyl, phenyl, an aromatic heterocycle, optionally pyridine, pyrrole, furan and imidazole, 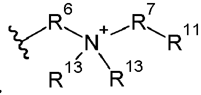 when forming R¹, 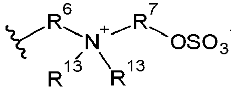 when forming R²;
   **R⁸** and **R⁹** together or two **R¹³** together optionally form an aliphatic or aromatic heterocycle, optionally a piperidine ring, a piperazine ring or a morpholine ring;
   **R⁸**, **R⁹** and **R⁶** or **R⁸**, **R⁹** and **R⁷** optionally together form a five or six-membered ring, optionally a pyridine ring, a piperidine ring, pyrrole ring, a pyrimidine ring, a pyrazole ring, an imidazole ring, a pyrazine ring, an isoxazole ring or an oxazole ring; wherein one of **R⁸** or **R⁹** is absent if **R⁸** and **R⁶**, **R⁹** and **R⁶** or **R⁸** and **R⁷,** or **R⁹** and **R⁷** together form an aromatic ring;
   **R¹¹** is selected from the group consisting of -SO3⁻, -C(=O)O⁻, and 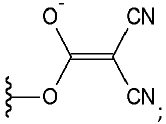
   **R¹²** is selected from the group consisting of methyl, ethyl and propyl;
   **R¹³** is independently selected from the group consisting of linear or branched, substituted or non-substituted (C₁₋₁₀)alkyl, optionally (C₁₋₅)alkyl, optionally methyl, ethyl and propyl, (C₂₋₁₀)alkenyl, phenyl, an aromatic heterocycle, optionally pyridine, pyrrole, furan and imidazole,
   wherein the polymer optionally is crosslinked by a crosslinker, optionally a crosslinker as R¹.

It is noted that the target binding moiety (TBM) as used herein does not necessarily refer to the target suitable for activating the Cas complex. Rather, the TBM can bind or attract any target of interest, including a Cas complex, a reporter, a cleaved detection moiety, a target suitable for activating the Cas complex or any other component of the composition or kit of parts disclosed herein depending on how the TBM is chosen and depending on the chemical properties of the components of the composition or kit of parts.

In an embodiment of the composition or kit of parts according to the present invention which may be combined with any of the embodiments of the composition or kit of parts preaddressed or still to be addressed unless in contradiction, the composition or kit of parts according to the present invention is one, wherein
**R¹** is selected from the group consisting of 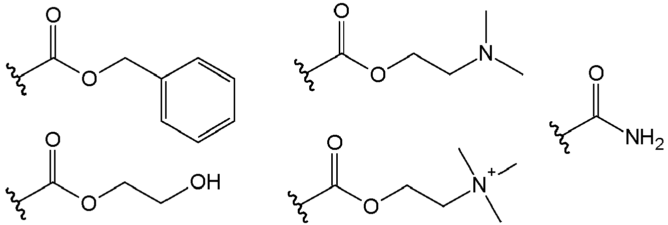 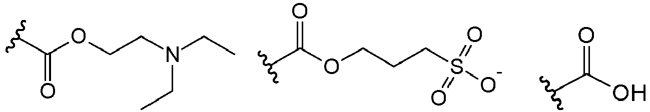 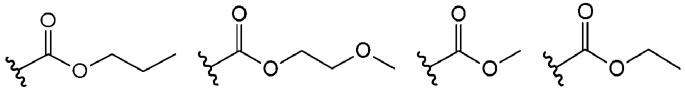 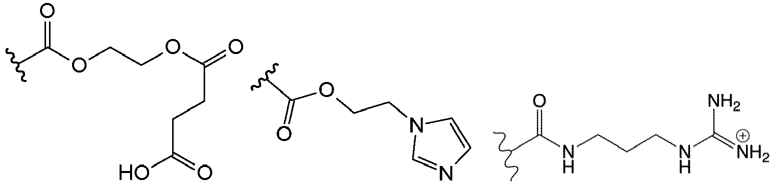 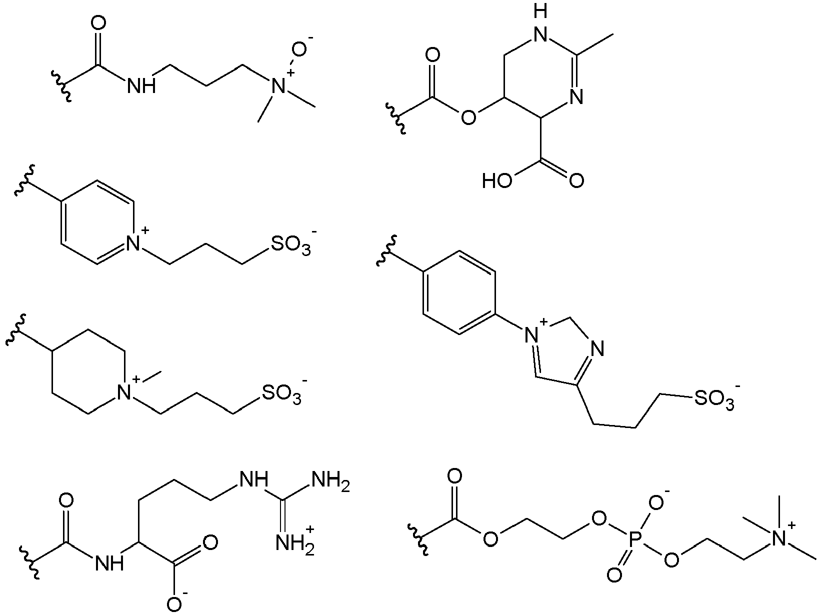 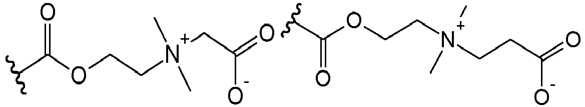 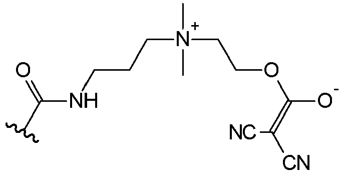 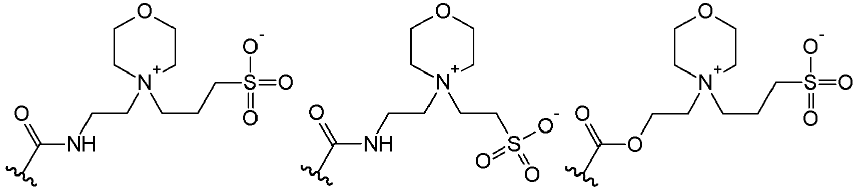 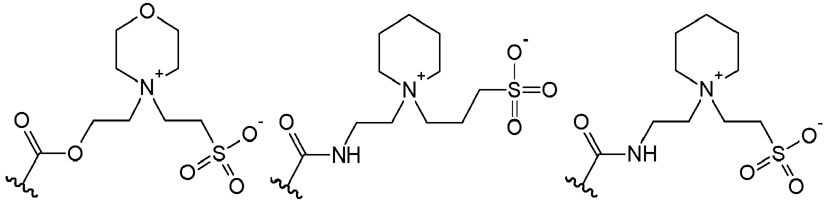 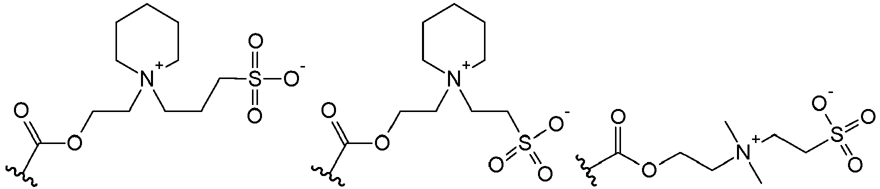 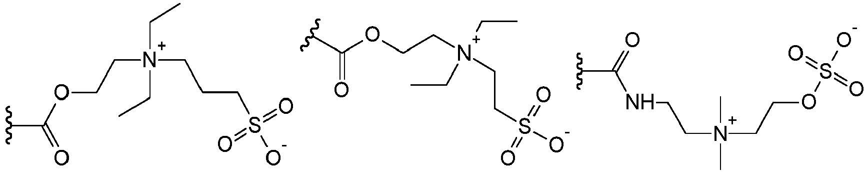 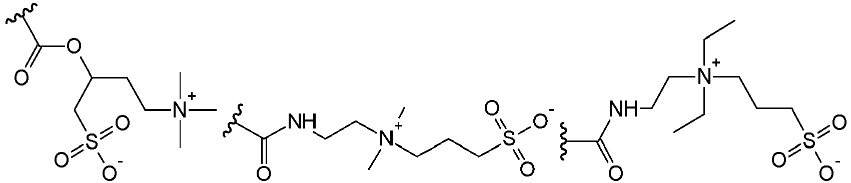 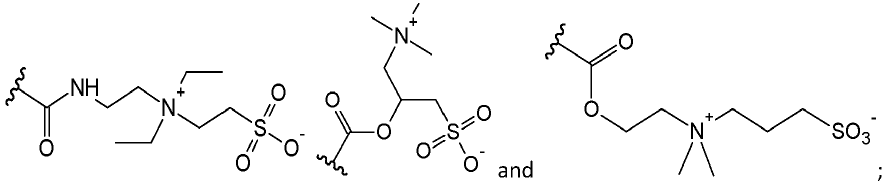 and
**R² is**

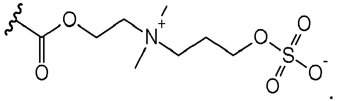

In an embodiment of the composition or kit of parts according to the present invention which may be combined with any of the embodiments of the composition or kit of parts preaddressed or still to be addressed unless in contradiction, at least one of R⁵ and R^{5'} is a TBM if
R¹ is selected from 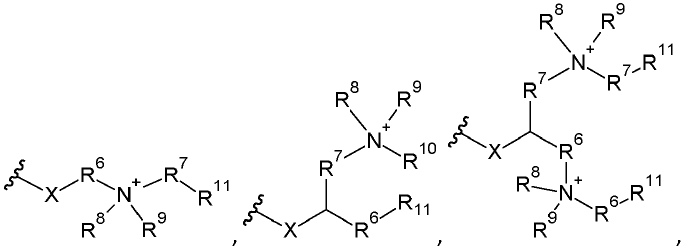 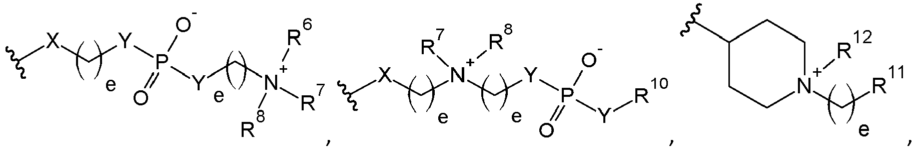 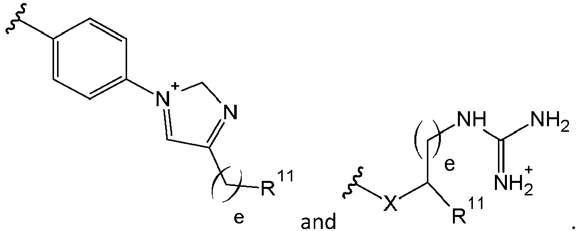 In this embodiment, it is evident that n is not zero if R¹ is present.

In the context of the present invention, the term "comprising a polymer according to formula (I)" means comprising at least one, i.e. one or more (different) polymers according to formula (I).

The polymers, e.g. according to Formula (I), of the droplets described herein for all aspects and embodiments are able to encode electrostatic attractive interactions, leading to the formation of the liquid droplets with many relevant features, for example: i) the droplets present low interfacial tension and, for this reason, generally do not require a high amount of energy to be generated; ii) the droplets can reversibly assemble and disassemble upon changes in the surrounding environment (e.g. by changing either temperature or ionic strength); and iii) the droplets can avoid unspecific protein adsorption on their surface and optionally exclude many solutes. If more than one polymer is present in the liquid droplets, e.g. two different polymers according to formula (I) or one polymer according to formula (I) and another polymer according to a different formula, the different polymers may have different functions, e.g. one polymer may induce phase separation and the other polymer may change the surface properties of the liquid droplet or comprise a component (e.g. is covalently bound to), e.g. a Cas complex, of the present composition or kit of parts. When functionalized with a target binding moiety (TBM, e.g. antibodies, biotin, etc. which can be easily conjugated to the polymer as R⁵ and/or R^{5'}), the droplets can selectively interact with a molecule of interest, e.g. with a Cas complex, a reporter, a (cleaved) detection moiety, or a target suitable for activating the Cas complex, and preferentially exclude all other components. The droplets can be therefore used to, e.g., upconcentrate and separate only the desired components from complex mixtures. Also, the targeting binding moiety can aid in an upconcentration which goes beyond the statistical distribution of compounds of interest. The liquid nature of the droplet (and optionally the spherical shape, i.e. high surface to volume ratio) is an important aspects to increase the solute upconcentratrion due to the presence of a dynamic interface. Moreover, the possibility to choose different target binding moieties confers modularity and flexibility. Additionally, the technology involving the droplets described herein is also easily scalable.

The notation of Formula (I) 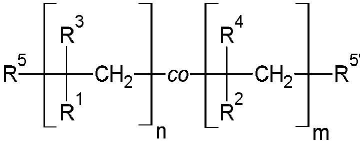 and any other formula herein which discloses a polymer by means of n- and m-numbers of building blocks, is to be understood as follows. The "co", as defined in IUPAC nomenclature, means that the sequence of monomers within the polymer is not specified, i.e. there is an unspecified sequence of monomers in the polymer. Optionally, the distribution of the monomers can be alternating, statistical, or in the form of a block co-polymer. R⁵ and R^{5'} on either end (alpha and omega end) of the polymer can be a chemical entity or group resulting from the reaction of a polymerization initiator, which may be further functionalized by a TBM. Exemplary chemical entities resulting from polymerization reactions include, e.g., groups resulting from a reversible addition-fragmentation chain transfer (RAFT) reagent, an atom transfer radical polymerization (ATRP) reagent or free-radical polymerization (FRP) reagent. These reagents and the products of the reagents that are formed as groups on the polymer ends are well known in the art. For example, RAFT reagents are commercially available trithiocarbonates, dithiocarbamates, dithiobenzoates, switchable RAFT reagents or macro-RAFT reagents, e.g. as available from Sigma Aldrich Canada Co. or Merck KGaA, Darmstadt, Germany. It is within the skilled person's general chemical knowledge what the structure of R⁵ and/or R^{5'} will be when choosing a specific polymerization initiator. Alternatively, R⁵ and/or R^{5'} may be a chemical entity resulting from a polymerization reaction as described herein which entity is further chemically modified (e.g. reacted with a TBM) to be a target binding moiety, or R⁵ and/or R^{5'} may be a target binding moiety in the absence of a chemical entity resulting from a polymerization. The term "a TBM attached to or reacted with a group resulting from a polymerization initiator" means that a TBM is attached to the group by means of a chemical reaction to form, e.g. a covalent or ionic, bond with the chemical entity resulting from a polymerization. This attachment may result in the direct attachment of the TBM to the polymer under loss of the chemical entity resulting from a polymerization or it may result in the chemical modification of the chemical entity, e.g. partial loss of chemical entity.

In any case, the target binding moiety is optionally selected from the group consisting of azide, alkyne, carboxylate, amine, thiol, hydroxyl, aldehyde, cyanate, sulfonyl, tosyl, tresyl, epoxide, carbonate, anhydride, carbamate, imidazole, azlactone, triazine, maleimide, aziridine, peroxide, acyl, anthraquinone, diazo, diazirine, psoralen, NHS ester, imido ester, (C₂₋₁₀)alkenyl or alkyl groups, DBCO, cytosine, guanidine, streptavidin, avidin, biotin, an aptamer, a peptide, a protein, an oligonucleotide, a nucleic acid, a carbohydrate, a dye, a cell, an antibody, an organic and inorganic nanoparticle, positively and negatively charged oligopolymers, a molecularly imprinted polymer, and an affinity polymer.

The polymer according to Formula (I) described herein for all aspects and embodiments may, optionally, be crosslinked by any suitable crosslinker. A crosslinker is a chemical agent that crosslinks the polymer, i.e. binds together two or more polymer chains, by attaching via at least two moieties of the same crosslinker to two or more polymer chains. The crosslinking can be reversible or irreversible and it can be based on a chemical linkage or on non-covalent interactions between the crosslinker and the polymer. Suitable crosslinkers for polymers are known to the skilled person and include but are not limited to, e.g. bismethacrylates, bismethacrylamides, and glutharaldeyde. Examples of non-covalent crosslinkers include, e.g., streptavidin that is able to bind with biotin-conjugated polymers or nanoparticles/proteins/compounds decorated with multiple antibodies or targeting agents.

The target binding moiety (TBM), as used herein for all aspects and embodiments, includes any suitable chemical or biological moiety that can bind a desired target, e.g. a Cas complex, a reporter, a (cleaved) detection moiety or a target suitable for activating the Cas complex to be taken up by the droplets, reversibly or irreversibly, covalently or non-covalently. Optionally, the target binding moiety is chosen such that it binds only to the desired component when in use together as part of the liquid droplets described herein. Examples for reactive groups or moieties for bioconjugations are known in the art (see, e.g., Greg T. Hermanson, Chapter 3 - The Reactions of Bioconjugation, Bioconjugate Techniques (Third Edition), 2013, Pages 229-258). For example, the TBM can be 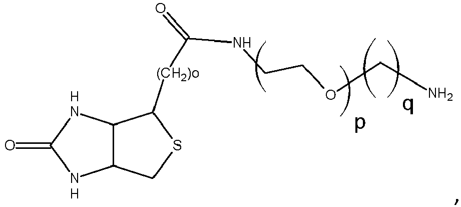 wherein o in (CH₂)o is an integer from 1 to 10, optionally 2 to 5, p is an integer from 1 to 5, optionally 2, and q is an integer from 1 to 5, and the TBM is connected via the amine to the polymer directly or to the entity resulting from polymerization described herein. Optionally, the TBM can be 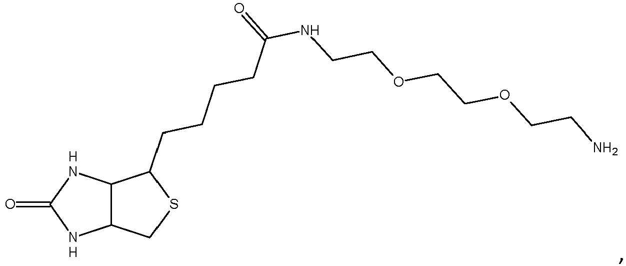 attached via the amine to the polymer according to Formula (I) described herein, optionally to R⁵ or R^{5'} being 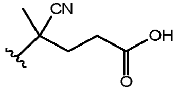 and from an amide bond.

The term DBCO, as used herein, refers to dibenzocyclooctyne, which is useful, e.g., in strain-promoted copper-free azide-alkyne cycloaddition reactions.

An antibody, as used herein for all aspects and embodiments, includes functional fragments and functional derivatives thereof or antibody-like binding proteins that bind a desired target, e.g. a compound to be taken up by the droplets. These are routinely available by hybridoma technology (Kohler and Milstein, Nature 256, 495-497, 1975), antibody phage display (Winter et al., (1994) Annu. Rev. Immunol. 12, 433-455), ribosome display (Schaffitzel et al., (1999) J. Immunol. Methods, 231, 119-135) and iterative colony filter screening (Giovannoni et al., (2001) Nucleic Acids Res. 29, E27) once the desired target is available. Typical proteases for fragmenting antibodies into functional products are well-known. Other fragmentation techniques can be used as well as long as the resulting fragment has a specific high affinity and, preferably a dissociation constant in the micromolar to picomolar range. A very convenient antibody fragment for targeting applications is the single-chain Fv fragment, in which a variable heavy and a variable light domain are joined together by a polypeptide linker. Other antibody fragments for target binding include Fab fragments, Fab₂ fragments, miniantibodies (also called small immune proteins), tandem scFv-scFv fusions as well as scFv fusions with suitable domains (e.g. with the Fc portion of an immune-globulin). For a review on certain antibody formats, see Holliger P, Hudson PJ.; Engineered antibody fragments and the rise of single domains. Nat Biotechnol. 2005 Sep., 23(9):1126-36). The term "functional derivative" of an antibody for use in the present invention is meant to include any antibody or fragment thereof that has been chemically or genetically modified in its amino acid sequence, e.g. by addition, substitution and/or deletion of amino acid residue(s) and/or has been chemically modified in at least one of its atoms and/or functional chemical groups, e.g. by additions, deletions, rearrangement, oxidation, reduction, etc. as long as the derivative has substantially the same binding affinity as to its original antigen and, optionally, has a dissociation constant in the micro-, nano- or picomolar range. The antibody, fragment or functional derivative thereof for use in the present invention can be, for example, one that is selected from the group consisting of polyclonal antibodies, monoclonal antibodies, chimeric antibodies, humanized antibodies, CDR-grafted antibodies, Fv-fragments, Fab-fragments and Fab₂-fragments and antibody-like binding proteins, e.g. affilines, anticalines and aptamers, any protein-ligand binding pair and the fragments of the both. For a review of antibody-like binding proteins see Binz et al. on engineering binding proteins from non-immunoglobulin domains in Nature Biotechnology, Vol. 23, No. 10, October 2005, 12571268. The term "aptamer" describes nucleic acids that bind to a polypeptide with high affinity. Aptamers can be isolated from a large pool of different single-stranded RNA molecules by selection methods such as SELEX (see, e.g., Jayasena, Clin. Chem., 45, p. 1628 - 1650, (1999); Klug and Famulok, M. Mol. Biol. Rep., 20, p. 97 - 107 (1994); US 5,582,981). Aptamers can also be synthesized and selected in their mirror form, for example, as the L-ribonucleotide (Nolte et al., (1996) Nat. Biotechnol., 14, pp.1116 - 1119; Klussmann et al., (1996) Nat. Biotechnol. 14, p. 1112 - 1115). Forms isolated in this way have the advantage that they are not degraded by naturally occurring ribonucleases and, therefore, have a greater stability. Another antibody-like binding protein and alternative to classical antibodies are the so-called "protein scaffolds", for example, anticalines, that are based on lipocaline (Beste et al., (1999) Proc. Natl. Acad. Sci. USA, 96, p. 1898 - 1903). The natural ligand binding sites of lipocalines, for example, of the retinol-binding protein or bilin-binding protein, can be changed, for example, by employing a "combinatorial protein design" approach, and in such a way that they bind selected haptens (Skerra, (2000) Biochem. Biophys. Acta, 1482, pp. 337 - 350). For other protein scaffolds it is also known that they are alternatives for antibodies (Skerra, (2000) J. Mol. Recognit, 13, pp. 167 - 287; Hey, (2005) Trends in Biotechnology, 23, pp. 514-522). In summary, the term functional antibody derivative is meant to include the above protein-derived alternatives for antibodies, i.e. antibody-like binding proteins, e.g. affilines, anticalines and aptamers that specifically recognize a desired target, compound, polypeptide, fragment or derivative thereof.

Examples for dyes, as used herein for all aspects and embodiments include, e.g., ATTO-488, ATTO390, ATTO647N, ATTO565, ALEXA488, Rhodamine B, Fluorescein, Amplex Red, fluorescamine, aromatic dialdehyde naphthalene-2,3-dicarboxaldehyde (NDA), or fluoraldehyde.

Examples for organic and inorganic nanoparticles, as used herein for all aspects and embodiments include, e.g., magnetic nanoparticles, quantum dots, metal-organic framework (MOF) nanoparticles.

Examples for positively and negatively charged oligopolymers, as used herein for all aspects and embodiments, include, e.g., poly(methacrylic acid sodium salt), poly(2-dimethylamino)ethyl methacrylate) methyl chloride quaternary salt, and sodium alginate.

Examples for carbohydrates include chitosan and heparin.

As used herein, a molecularly imprinted polymer is a polymer that can selectively bind the product with which it was imprinted during production. Examples for molecularly imprinted polymers are imprinted acrylic and methacrylic polymers (BelBruno, (2019), Chem. Rev., 119, 1, pp. 94-119).

Affinity polymers include, e.g., epitope-selective linear copolymers (Latza, (2014), Chem. Eur. J., 20, pp. 11479-11487).

As used herein for all aspects and embodiments, a "substituent" or "residue" or **"R",** refers to a molecular moiety that is covalently bound to an atom within a molecule of interest. For example, a "substituent", **"R"** or "residue" may be a moiety such as a halogen, alkyl group, haloalkyl group or any other substituent described herein that is covalently bonded to an atom, optionally a carbon, oxygen or nitrogen atom, that forms part of a molecule of interest.

The group **X** used herein for all aspects and embodiments is selected from the group consisting of -C(=O)-O-, -C(=O)-NH-, aliphatic and aromatic carbo- or heterocycle. In any aspect disclosed herein, the group "-C(=O)-O-" or "-C(=O)-NH-" is annotated such that the attachment point (indicated by the waved line) of the moiety comprising said group is at the beginning of either -C(=O)-O- or -C(=O)-NH-. In other words and as a representative example for all aspects and embodiments, if **X** is selected to be -C(=O)-O- or -C(=O)-NH- in a residue of the following formula: 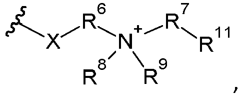 the following structures result: 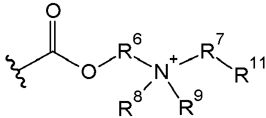 or

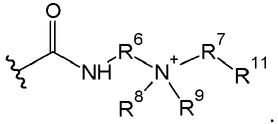

The term "non-substituted" as used herein shall mean substituted only with hydrogen. The term "substituted" as used herein, means that any one or more hydrogens on the designated atom or group is replaced, independently, with an atom different from hydrogen, optionally by a halogen, optionally by fluorine, a thiol, a carboxyl, a cyano, a nitro, an alkyl (optionally C₁-C₁₀), aryl (optionally phenyl, benzyl or benzoyl), an alkoxy group, a sulfonyl group, by a tertiary or quaternary amine or by a selection from the indicated substituents, provided that the designated atom's normal valence is not exceeded, and that the substitution results in a stable compound, i.e., a compound that can be isolated and characterized using conventional means.

The term "heteroatom" as used herein shall be understood to mean atoms other than carbon and hydrogen such as, e.g., O, N, S and P.

In the context of the present invention, it is understood that antecedent terms such as "linear or branched", "substituted or non-substituted" indicate that each one of the subsequent terms is to be interpreted as being modified by said antecedent term. For example, the scope of the term "linear or branched, substituted or non-substituted alkyl, alkenyl, alkynyl, carbocycle" encompasses linear or branched, substituted or non-substituted alkyl; linear or branched, substituted or non-substituted alkenyl; linear or branched, substituted or non-substituted alkynyl; linear or branched, substituted or non-substituted alkylidene; and linear or branched, substituted or non-substituted carbocycle. For example, the term "(C₂₋₁₀) alkenyl, alkynyl or alkylidene" indicates the group of compounds having 2 to 10 carbons and alkenyl, alkynyl or alkylidene functionality.

The expression "alkyl" refers to a saturated, straight-chain or branched hydrocarbon group that contains the number of carbon items indicated, e.g. linear or branched "(C₁₋₁₀)alkyl" denotes a hydrocarbon residue containing from 1 to 10 carbon atoms, e.g. a methyl, ethyl, propyl, iso-propyl, n-butyl, iso-butyl, sec-butyl, tert-butyl, n-pentyl, iso-pentyl, n-hexyl, 2,2-dimethylbutyl, etc.

The expression "alkenyl" refers to an at least partially unsaturated, substituted or non-substituted straight-chain or branched hydrocarbon group that contains the number of carbon atoms indicated, e.g. "(C₂₋₁₀)alkenyl" denotes a hydrocarbon residue containing from 2 to 10 carbon atoms, for example an ethenyl (vinyl), propenyl (allyl), iso-propenyl, butenyl, isoprenyl or hex-2-enyl group, or, for example, a hydrocarbon group comprising a methylene chain interrupted by one double bond as, for example, found in monounsaturated fatty acids or a hydrocarbon group comprising methylene-interrupted polyenes, e.g. hydrocarbon groups comprising two or more of the following structural unit -[CH=CH-CH₂]-, as, for example, found in polyunsaturated fatty acids. Alkenyl groups have one or more, e.g. 1, 2, 3, 4, 5, or 6 double bond(s).

The expression "alkyne" refers to at least partially unsaturated, substituted or non-substituted straight-chain or branched hydrocarbon groups that may contain, e.g. from 2 to 10 carbon atoms, for example an ethinyl, propinyl, butinyl, acetylenyl, or propargyl group. Optionally, alkine groups have one or two (e.g. one) triple bond(s).

Furthermore, the terms "alkyl", "alkenyl" and "alkyne" also refer to groups in which one or more hydrogen atom(s) have been replaced, e.g. by a halogen atom, optionally F, Cl or Br, such as, for example, a 2,2,2-trichloroethyl, tribromoethyl or a trifluoromethyl group.

The term "heterocycle" refers to a stable substituted or non-substituted, aromatic or nonaromatic, optionally 3 to 10 membered, optionally 3-6 membered, optionally 5 or 6 membered, monocyclic, heteroatom-containing cycle. Each heterocycle consists of carbon atoms and one or more, optionally 1 to 4, optionally 1 to 3 heteroatoms optionally chosen from nitrogen, oxygen and sulphur. A heterocycle may contain the number of carbon atoms in addition to the non-carbon atoms as indicated: a "(C₃₋₆)heterocycle" is meant to have 3 to 6 carbon atoms in addition to a given number of heteroatoms.

Exemplary heterocycles include, but are not limited to pyridine, piperidine, pyrrole, pyrimidine, pyrazole, imidazole, imidazole, pyrazine, isoxazole or oxazole.

The expressions "alkyl ether" refers to a saturated or non-saturated, straight-chain or branched hydrocarbon group that contains the number of carbon items indicated. For example, "(C₂₋₅)alkyl ether" denotes a hydrocarbon residue containing from 2 to 5 carbon atoms, and any suitable number of oxygen atoms that will result in an ether structure. Alkyl ether groups as used herein, shall be understood to mean any linear or branched, substituted or non-substituted alkyl chain comprising an oxygen atom either as an ether motif, i.e. an oxygen bound by two carbons. Exemplary alkyl ethers are polyethylene glycol (PEG) chains. The term polyethylene glycol as used herein refers to a chain of substituted or non-substituted ethylene oxide monomers. The ether residue can be attached to the Formula provided in the present invention either via the carbon atom or via the oxygen atom of the ether residue.

If more than one residue **R** is attached to a given atom of a formula described herein, one residue **R** can be absent if the attachment of the other **R** leads to a full valency of the atom. For example, **R⁸** or **R⁹** is absent if **R⁸** and **R⁶**, **R⁹** and **R⁶** or **R⁸** and **R⁷**, or **R⁹** and **R⁷** together form an aromatic ring.

As used herein, a wording defining the limits of a range of length such as, e. g., "from 1 to 5" or "(C₁₋₅)" means any integer from 1 to 5, i.e. 1, 2, 3, 4 and 5. In other words, any range defined by two integers explicitly mentioned is meant to comprise and disclose any integer defining said limits and any integer comprised in said range.

The scope of the present invention includes those analogs of the compounds as described above and in the claims that feature the exchange of one or more carbon-bonded hydrogens, optionally one or more aromatic carbon-bonded hydrogens, with halogen atoms such as F, Cl, or Br, optionally F. The exchange of one or more of the carbon-bonded hydrogens, e.g. by fluorine, can be done, e.g., for reasons of metabolic stability and/or pharmacokinetic and physicochemical properties.

In an embodiment of the composition or kit of parts according to the present invention which may be combined with any of the embodiments of the composition or kit of parts preaddressed or still to be addressed unless in contradiction, the composition or kit of parts of the present invention is one, wherein the polymer according to Formula (I) is selected from the group consisting of 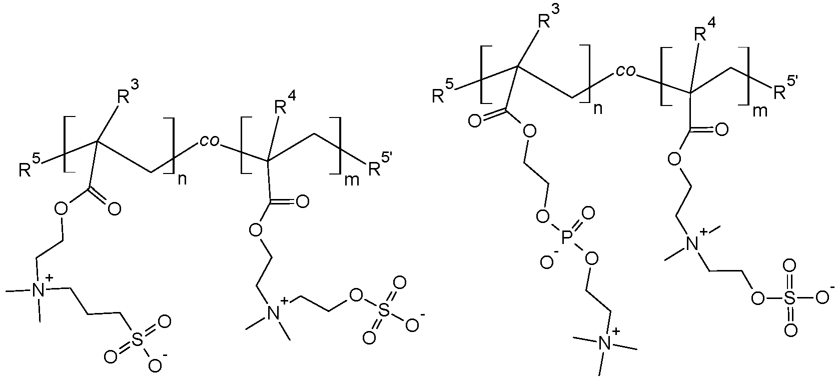 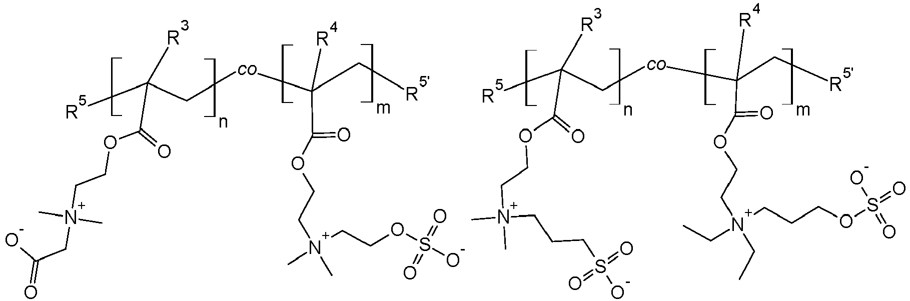 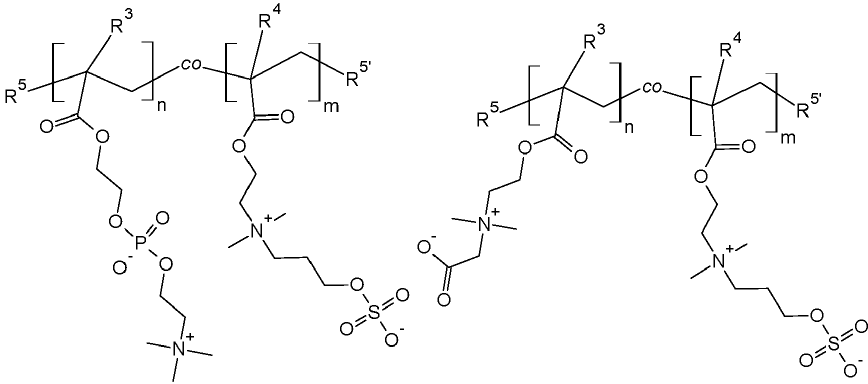 and

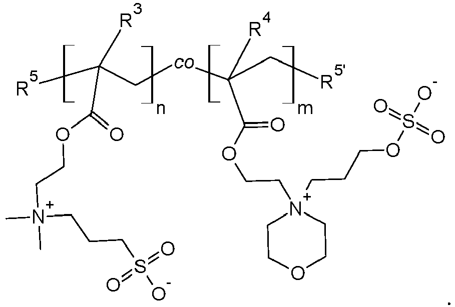

Optionally, R⁵ and R^{5'} of the above-noted polymers are selected from a group resulting from a RAFT polymerization initiator, optionally 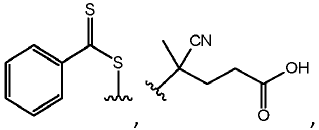 or a target binding moiety (TBM), optionally a TBM attached to or reacted with a group resulting from a polimerization initiator, optionally from a RAFT polymerization initiator, optionally 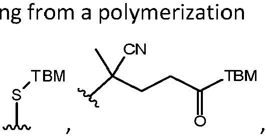 and 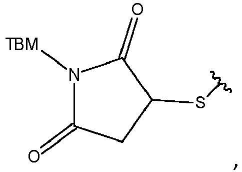 optionally 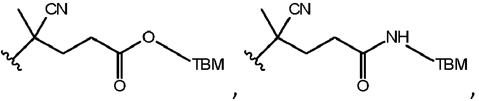 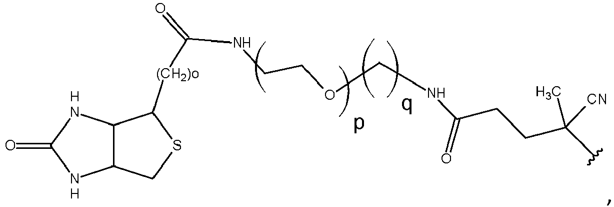 and 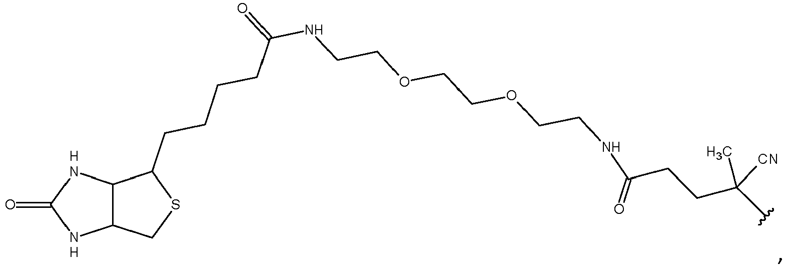 wherein o in (CH₂)o is an integer from 1 to 10, optionally 2 to 5, p is an integer from 1 to 5, optionally 2, and q is an integer from 1 to 5.

In an embodiment of the composition or kit of parts according to the present invention which may be combined with any of the embodiments of the composition or kit of parts preaddressed or still to be addressed unless in contradiction, the composition or kit of parts of the present invention is one, wherein the crosslinker for polymer according to Formula (I) is selected from the group consisting of 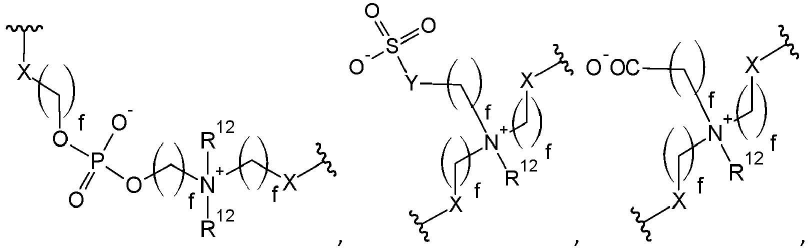 and 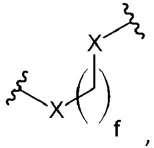 wherein
**f** is an integer selected from 1 to 5, optionally from 2 to 3;
**X** is selected from the group consisting of -C(=O)-O-, -C(=O)-NH-, aliphatic and aromatic carbo- or heterocycle, optionally -C(=O)-O- and -C(=O)-NH-;
**Y** is oxygen or absent; and
**R¹²** is selected from the group consisting of methyl, ethyl and propyl;
optionally the crosslinker is selected from the group consisting of

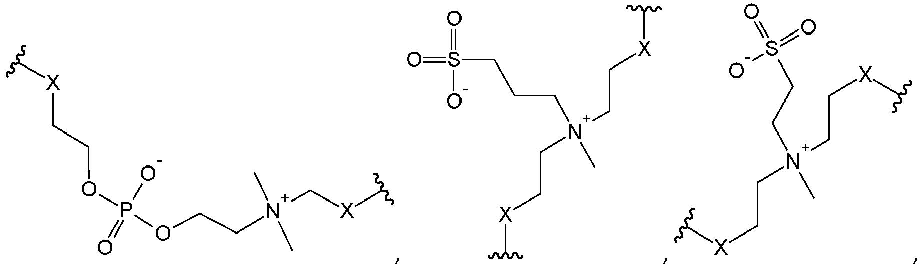

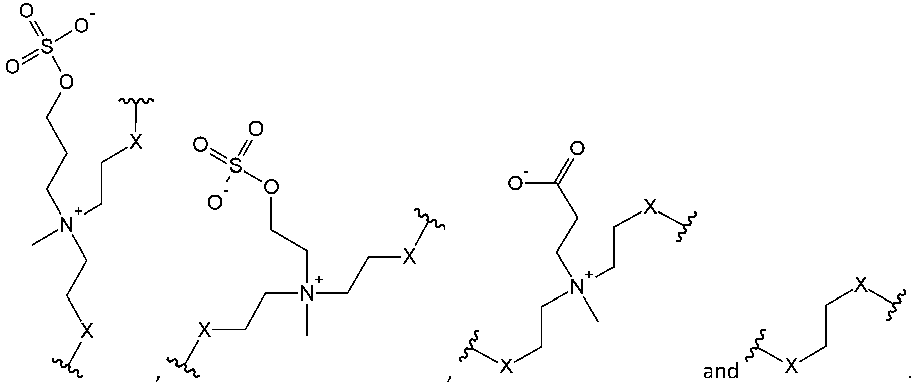

The crosslinker is attached to the backbone of the polymer at the position indicated with the wavy line. The crosslinker can, e.g., crosslink two R¹ or R² positions or an R¹ with an R² position, or an R² with an R¹ position in the polymer backbone.

Optionally,
a. the polymer according to Formula (I) has a degree of polymerization (n+m) from 5 to 5000, optionally from 50 to 500; and/or
b. the percentage of n in the polymer according to Formula (I) ([n/(n+m)]^{∗}100) is in the range from 0 to 99.5%, optionally 0 to 70%, optionally 20 to 60%;
c. the dispersity of the polymer according to Formula (I) is in the range of 1 to 5, optionally 1 to 1.5;
d. the crosslinker molar fraction in the polymer according to Formula (I) is in the range of 0 to 99%, optionally 0 to 5%; and/or
e. the coacervates formed by the polymer according to Formula (I) are responsive to temperature, shear, ionic strength, pH and/or a magnetic field.

The degree of polymerization and the dispersity can be determined by known methods. For example, nuclear magnetic resonance (¹H-NMR) can be used for determining the degree of polymerization and polymer dispersity and molecular weight can be evaluated via gel permeation chromatography.

Responsiveness to temperature, ionic strength, pH, shear and/or a magnetic field means, for example, that the droplets can be disassembled and/or assembled upon change of temperature, ionic strength and/or pH. The morphology of the droplets can be changed, e.g., by applying a magnetic field. Moreover, the surface tension, multiphase miscibility, size, polarity, viscosity, water content, and/or density of the droplets can be controlled, e.g., by changing the properties of the surrounding environment (e.g. temperature, ionic strength, pH, shear, and composition). This control and responsiveness also allows for the control of the uptake of compounds from outside to the inside of the liquid droplets as described above.

In an embodiment of the composition or kit of parts according to the present invention which may be combined with any of the embodiments of the composition or kit of parts preaddressed or still to be addressed unless in contradiction, the composition or kit of parts of the present invention is one, wherein the polymer according to Formula (I) is 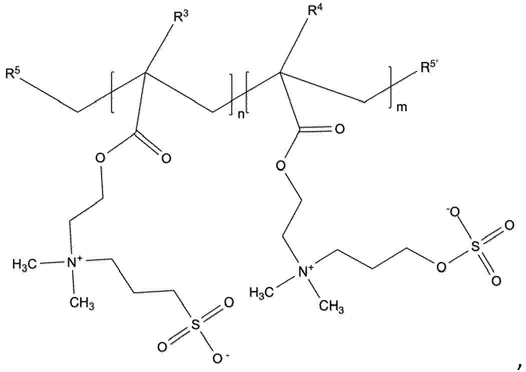 wherein **m** is an integer from 100 to 800, **n** is selected so that n/(n+m)^{∗}100 is between 0.125% and 50% and **R^{5'}** and/or **R⁵** is a TBM, optionally a protein, a peptide, or an aptamer; or wherein **m** is an integer from 70 to 100, **n** is selected so that n/(n+m)^{∗}100 is between 1% and 30% and **R^{5'}** and/or **R⁵** is a TBM, optionally a protein, a peptide, or an aptamer.

In an embodiment of the composition or kit of parts according to the present invention which may be combined with any of the embodiments of the composition or kit of parts preaddressed or still to be addressed unless in contradiction, the composition or kit of parts of the present invention is one, wherein the polymer according to Formula (I) is 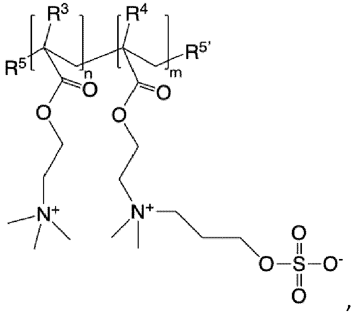 wherein **m** is an integer from 160 to 800, **n** is selected so that n/(n+m)^{∗}100 is between 0.125% and 40% and optionally **R^{5'}** and/or **R⁵** is a TBM, optionally a protein, a peptide, or an aptamer; or wherein **m** is an integer from 80 to 159, **n** is selected so that n/(n+m)^{∗}100 is between 0.625% and 20% and optionally **R^{5'}** and/or **R⁵** is a TBM, optionally a protein, a peptide, or an aptamer.

In an embodiment of the composition or kit of parts according to the present invention which may be combined with any of the embodiments of the composition or kit of parts preaddressed or still to be addressed unless in contradiction, the composition or kit of parts of the present invention is one, wherein the polymer according to Formula (I) is 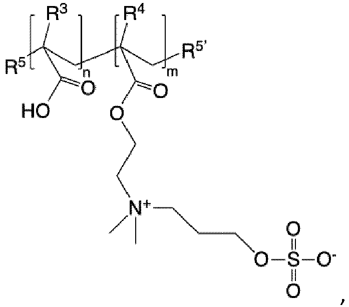 wherein **m** is an integer from 80 to 800, **n** is selected so that n/(n+m)^{∗}100 is between 0.125% and 20% and optionally **R^{5'}** and/or **R⁵** is a TBM, optionally a protein, a peptide, or an aptamer.

In an embodiment of the composition or kit of parts according to the present invention which may be combined with any of the embodiments of the composition or kit of parts preaddressed or still to be addressed unless in contradiction, the composition or kit of parts of the present invention is one, wherein the polymer according to Formula (I) is 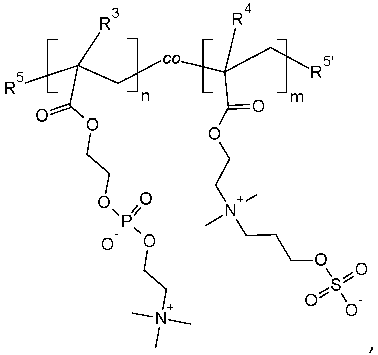 wherein **m** is an integer from 80 to 800, **n** is selected so that n/(n+m)^{∗}100 is between 0.125% and 15% and **R^{5'}** and/or R⁵ is a TBM, optionally a protein, a peptide, an aptamer, or a group resulting from a RAFT polymerization initiator, optionally

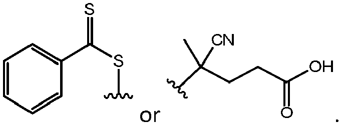

In an embodiment of the composition or kit of parts according to the present invention which may be combined with any of the embodiments of the composition or kit of parts preaddressed or still to be addressed unless in contradiction, the composition or kit of parts of the present invention is one, wherein the polymer according to Formula (I) is one, wherein **R¹** is 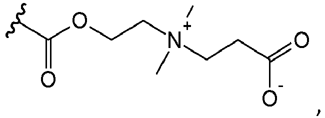 **R²** is 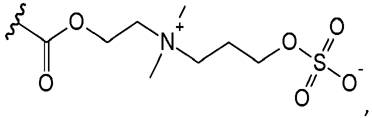 **m** is an integer from 160 to 800, **n** is selected so that n/(n+m)^{∗}100 is between 0.125% and 20% and **R^{5'}** and/or **R⁵** is a TBM, optionally a protein, a peptide, or an aptamer.

In an embodiment of the composition or kit of parts according to the present invention which may be combined with any of the embodiments of the composition or kit of parts preaddressed or still to be addressed unless in contradiction, the composition or kit of parts of the present invention is one, wherein the polymer according to Formula (I) is one, wherein R¹ is 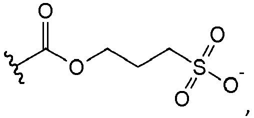 **R²** is 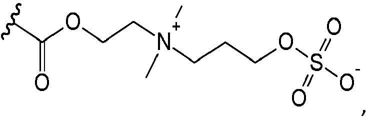 **m** is an integer from 20 to 800, **n** is selected so that n/(n+m)^{∗}100 is between 0.125% and 80% and optionally **R^{5'}** and/or **R⁵** is a TBM, optionally a protein, a peptide, or an aptamer.

In an embodiment of the composition or kit of parts according to the present invention which may be combined with any of the embodiments of the composition or kit of parts preaddressed or still to be addressed unless in contradiction, the composition or kit of parts of the present invention is one, wherein the polymer according to Formula (I) is one, wherein **R¹** is 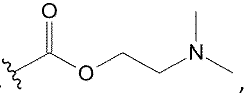 **R²** is 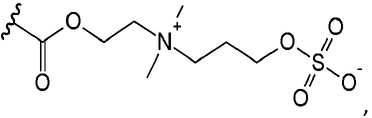 **m** is an integer from 80 to 800, **n** is selected so that n/(n+m)^{∗}100 is between 0.125% and 20% and optionally **R^{5'}** and/or **R⁵** is a TBM, optionally a protein, a peptide, or an aptamer.

In an embodiment of the composition or kit of parts according to the present invention which may be combined with any of the embodiments of the composition or kit of parts preaddressed or still to be addressed unless in contradiction, the composition or kit of parts of the present invention is one, wherein the polymer according to Formula (I) is one, wherein **R¹** is a hydrophobic residue, optionally 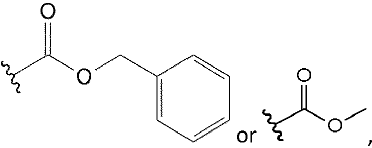 , **R²** is 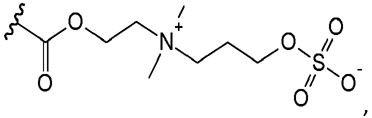 **m** is an integer from 1 to 900, **n** is optionally selected so that n/(n+m)^{∗}100 is between 0.125% and 20% and optionally **R^{5'}** and/or **R⁵** is a TBM, optionally a protein, a peptide, or an aptamer.

In an embodiment of the composition or kit of parts according to the present invention which may be combined with any of the embodiments of the composition or kit of parts preaddressed or still to be addressed unless in contradiction, the composition or kit of parts of the present invention is one, wherein the polymer according to Formula (I) is selected from the group consisting of: 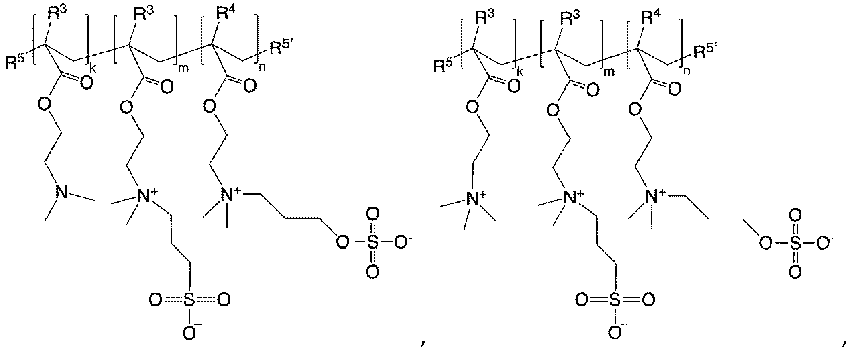 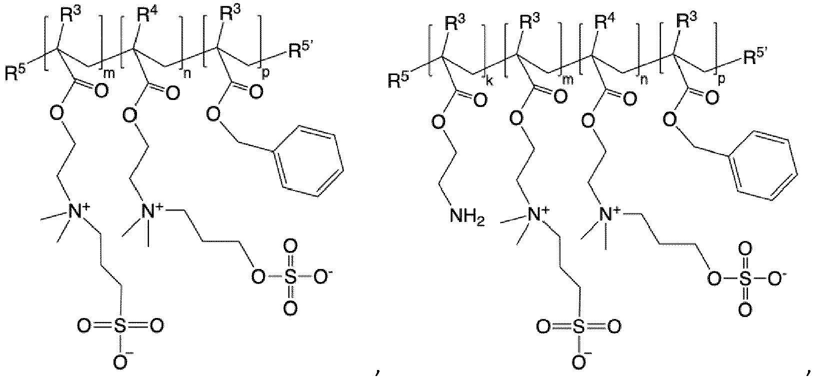 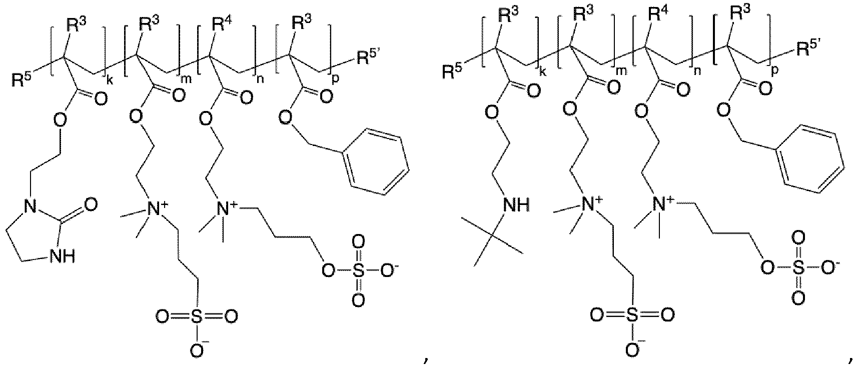 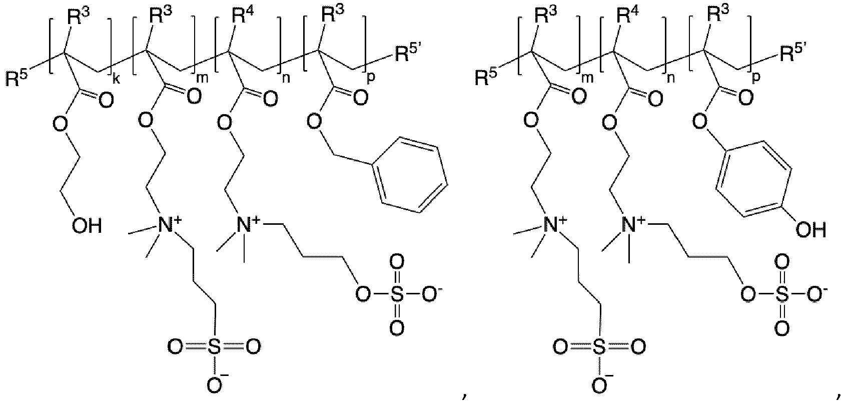 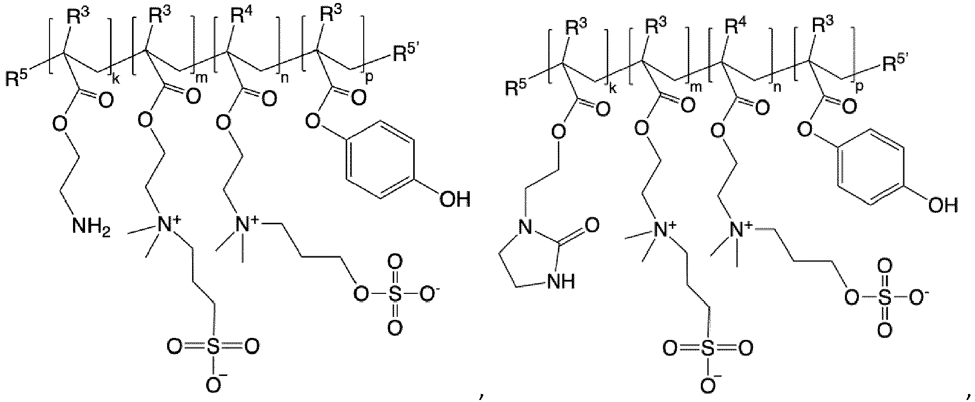 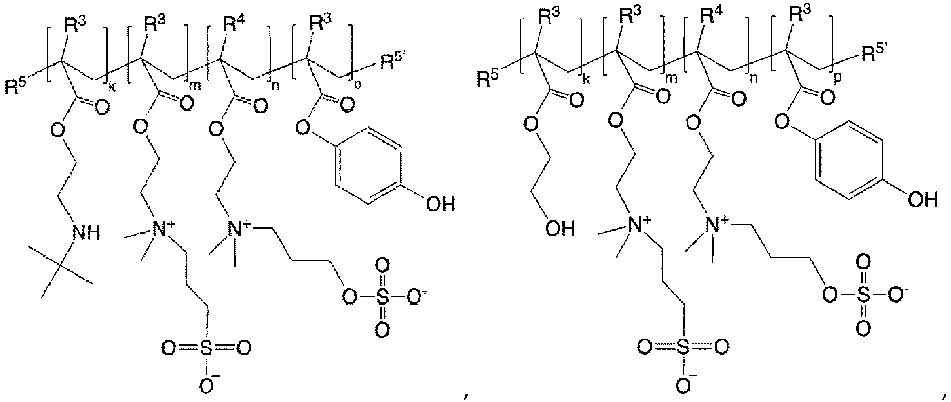 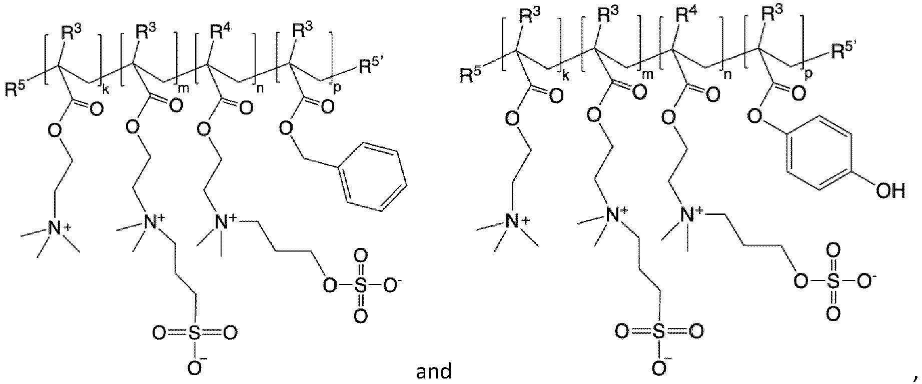 wherein **n** is an integer between 20 and 400, **k** is an integer selected so that k/(k+m+n+p)^{∗}100 is between 0.125% and 40%, **m** is an integer selected so that m/(k+m+n+p)^{∗}100 is between 0.125% and 80%, **p** is an integer selected so that p/(k+m+n+p)^{∗}100 is between 0.125% and 40%, **R³** and **R⁴** are each independently selected from the group consisting of hydrogen and methyl, and **R^{5'}** and/or **R⁵** is a TBM, optionally a protein, a peptide, or an aptamer.

For methods of preparing the polymers according to Formula (I), reference is made to WO2022/122675 which is incorporated herein in its entirety.

In an embodiment of the composition or kit of parts according to the present invention which may be combined with any of the embodiments of the composition or kit of parts preaddressed or still to be addressed unless in contradiction, the composition or kit of parts according to the present invention is one, wherein the Cas protein is selected from type I Cas, optionally Cas 3; type II Cas, optionally Cas 9; type III Cas, optionally Cas 10; type V Cas, optionally Cas 12 and Cas14; and type VI Cas, optionally Cas 13.

In an embodiment of the composition or kit of parts according to the present invention which may be combined with any of the embodiments of the composition or kit of parts preaddressed or still to be addressed unless in contradiction, the composition or kit of parts according to the present invention is one, wherein the Cas protein
a. is bound to a coacervate-forming compound as defined herein,
b. is bound, optionally covalently bound, at the N-terminus, C-terminus or both termini to an elastin-like polypeptide as defined herein,
c. is bound, optionally covalently bound, at the N-terminus, C-terminus or both termini to a low complexity domain (LCD) stretch from naturally occurring nucleic acid binding proteins as defined herein,
d. is bound, optionally covalently bound, at the N-terminus, C-terminus or both termini to a resilin-like polypeptide as defined herein,
e. has a TBM at least at the N-terminus, the C-terminus or both termini, optionally selected from the group consisting of
   (i) an amine group of the N-terminus or a carboxylate group of the C-terminus,
   (ii) a sulfhydryl group of cysteine, a guanidino group of arginine, a phenolate ring of tyrosine, an indole ring of tryptophan, a thioether ring of methionine, and an imidazole ring of histidine,
   (iii) an azide, DBCO, alkyne, alkene and acetyl group, optionally an azide or an alkyne group of a non-canonical amino acid,
   (iv) an affinity tag, optionally an alkaline phosphatase, AU1 epitope, AU5 epitope, T7-tag, V5-tag, biotin-carboxy carrier protein, B-tag, calmodulin binding peptide, chloroamphenicol acetyl transferase, cellulose binding domain, chitin binding domain, choline-binding domain, DHFR, E2 epitope, FLAG epitope, galactose binding protein, EE-tag, Glutathione S-transferase, Human influenza hemagglutinin, HaloTag, Histidine affinity tag, HSV epitope, Maltose-binding protein, Myc epitope, PDZ domain, polyhistidine, polyarginine, polyaspartic acid, polycystein, polyphenalanine, S-tag, streptavidin binding peptide, staphylococcal protein A, staphylococcal protein G, Strep-tag, Tandem affinity purification (TAP), T7 epitope, TrpE, and VSV-G, and
   (v) avidin, biotin, an aptamer, an oligonucleotide, a molecularly imprinted polymer, an affinity polymer, a carbohydrate, a dye, an organic or inorganic nanoparticle, and a positively or negatively charged polymer.

As exemplified above, the Cas protein can be, e.g., covalently bound, bound by affinity (electrostatic interactions or van der Waals forces), by host-guest interactions or complexation interactions to (a part of) the coacervate-forming compounds and the resulting coacervates. In the embodiment, in which the Cas protein is bound to an elastin-like polypeptide as defined herein, to a low complexity domain (LCD) stretch from naturally occurring nucleic acid as defined herein, or to a resilin-like polypeptide as defined herein, the elastin-like peptide, the LCD stretch and/or the resilin-like polypeptide form part of the coacervate-forming compounds and the coacervates resulting therefrom.

The Cas protein can have a TBM (covalently) bound to it. This TBM can bind to another TBM, e.g. another TBM bound to a coacervate-forming compound or bound to any other component of the composition or kit of parts described herein. The TBM can be bound to any of the components of the composition or kit of parts described herein directly or via suitable linker, e.g. a PEG linker. For example, the Cas protein can be expressed with any of (i) an amine group of the N-terminus or a carboxylate group of the C-terminus, (ii) a sulfhydryl group of cysteine, a guanidino group of arginine, a phenolate ring of tyrosine, an indole ring of tryptophan, a thioether ring of methionine, and an imidazole ring of histidine, or (iii) an azide, DBCO, alkyne, alkene and acetyl group, optionally an azide or an alkyne group, from a non-canonical amino acid. For example, the histidine can be further modified into an azide, DBCO, alkyne, alkene, and acetyl group or directly used to conjugate by click chemistry a polymer or an affinity tag, optionally avidin, biotin, an aptamer, an oligonucleotide, a molecularly imprinted polymer, an affinity polymer, a carbohydrate, a dye, an organic or inorganic nanoparticle, a positively or negatively charged polymer.

Also, and for example, the Cas protein can be expressed with an affinity tag, optionally an alkaline phosphatase, AU1 epitope, AU5 epitope, T7-tag, V5-tag, biotin-carboxy carrier protein, B-tag, calmodulin binding peptide, chloroamphenicol acetyl transferase, cellulose binding domain, chitin binding domain, choline-binding domain, DHFR, E2 epitope, FLAG epitope, galactose binding protein, EE-tag, Glutathione S-transferase, Human influenza hemagglutinin, HaloTag, Histidine affinity tag, HSV epitope, Maltose-binding protein, Myc epitope, PDZ domain, polyhistidine, polyarginine, polyaspartate, polycystein, polyphenalanine, S-tag, streptavidin binding peptide, staphylococcal protein A, staphylococcal protein G, Strep-tag, Tandem affinity purification (TAP), T7 epitope, TrpE, and VSV-G.

In an embodiment of the composition or kit of parts according to the present invention which may be combined with any of the embodiments of the composition or kit of parts preaddressed or still to be addressed unless in contradiction, the composition or kit of parts according to the present invention is one, wherein the reporter comprises a TBM, optionally an azide, an alkyne, a DBCO, a maleimide, a NHS ester, an epoxide, a biotin, a hydrophilic molecule, a charged molecule, a hydrophobic molecule, optionally cholesterol, digoxigenin, biotin, or a coacervate-forming compound as defined herein.

In an example, the TBM is added to the reporter on fluorophore or quencher sides, e.g. leading to the following exemplary structure: 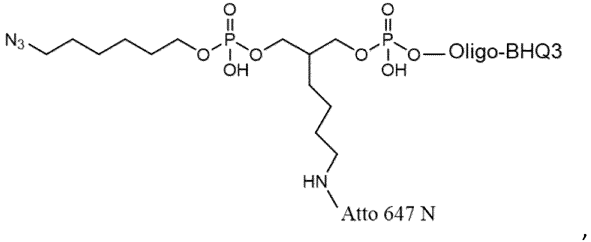 wherein N₃ is the TBM on the fluorophore side, ATTO647N the fluorophore, Oligo the ssDNA sequence and BHQ3 the quencher. For example, the reporter may comprise any of the TBMs described herein.

For example, the reporter can comprise a coacervate-forming compound which is a coacervate-forming as defined above, but which can be different compared to that of the coacervate-forming compounds used to recruit the target suitable for activating the Cas complex and the Cas protein. The functionalized reporter can interact with the coacervates that recruit a target suitable for activating the Cas complex and the Cas protein, or it can interact with other immiscible coacervates.

For example, the reporter can comprise a hydrophobic molecule such as cholesterol, biotin or digoxigenin adjacent to the fluorophore. The functionalized reporter cannot be taken up in hydrophobic coacervates due to the hydrophilicity of the nucleic acids. After cleavage by the Cas protein, the functionalized detection moiety can be taken up in hydrophobic coacervates because the length of the nucleic acid (hence, the hydrophilicity of the fluorophore side) is changed. The reporter can interact with the coacervates that recruit a target suitable for activating the Cas complex and the Cas protein, while the cleaved detection moiety can interact with other immiscible coacervates. An exemplary structure for a reporter comprising a hydrophobic molecule adjacent to the fluorophore and far from the quencher is shown below.

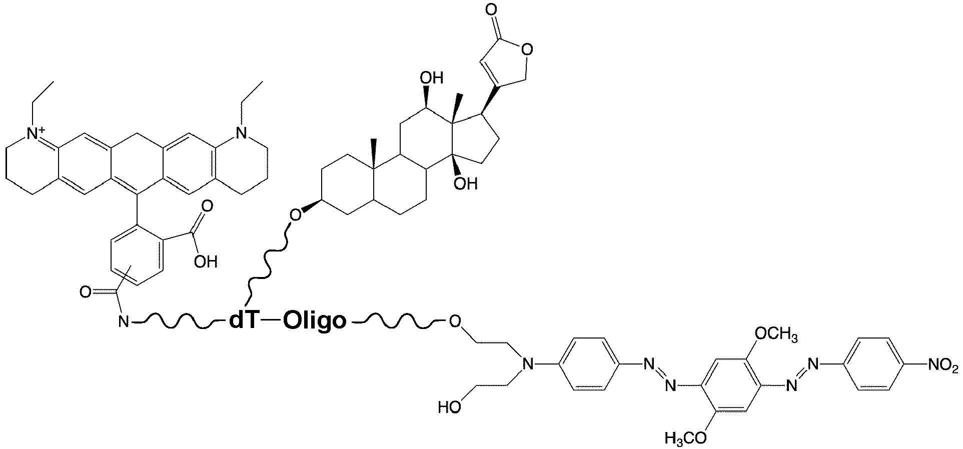

In an embodiment of the composition or kit of parts according to the present invention which may be combined with any of the embodiments of the composition or kit of parts preaddressed or still to be addressed unless in contradiction, the composition or kit of parts according to the present invention is one, wherein
A. the coacervate-forming compounds, the Cas complex and the reporter are configured such that the Cas complex, the reporter, the cleaved detection moiety and the optional target are taken up by coacervates formed by the coacervate-forming compounds, or
B. the coacervate-forming compounds, the Cas complex and the reporter are configured such that the Cas complex, the reporter and the optional target are taken up by coacervates formed by the coacervate-forming compounds, or
C. the coacervate-forming compounds, the Cas complex and the reporter are configured such that the cleaved detection moiety, optionally the fluorophore, is taken up by coacervates formed by the coacervate-forming compounds, or
D. the coacervate-forming compounds, the Cas complex and the reporter are configured such that the Cas complex and the optional target are taken up by coacervates formed by the coacervate-forming compounds, or
E. the coacervate-forming compounds, the Cas complex and the reporter are configured such that the Cas complex is located at an interphase between the coacervate and the outside of the coacervate, or
F. the coacervate-forming compounds, the Cas complex and the reporter are configured such that the Cas complex and the optional target are taken up by coacervates formed by the coacervate-forming compounds.

In the above embodiment (D), the reporter is optionally not accumulated in the coacervate, but it can still diffuse in and out of the coacervate.

In the above embodiment (E), the optional target and the reporter are optionally not accumulated in the coacervate, but it can still diffuse in and out of the coacervate.

In the above embodiment (F), the reporter is optionally not accumulated in the coacervate, but it can still diffuse in and out of the coacervate. After cleavage, the cleaved detection moiety (e.g. the fluorophore) can be, e.g., taken up in a separate third phase.

Exemplary interactions of the components of the present composition or kit of parts are provided below.

### Reaction components

-Cas complex: Cas complex comprising a guide RNA and a Cas protein as defined herein, wherein the Cas protein has trans or collateral cleavage activity for a nucleic acid sequence of a reporter;
- target: target suitable for activating the Cas complex;
- reporter: reporter comprising a detection moiety covalently linked to the nucleic acid sequence,
- coacervate-forming compound

### Interaction combination table

| Reaction Component | Cas complex | target | reporter | coacervate-forming compound |
|---|---|---|---|---|
| Cas complex | (1,1) | (1,2) | (1,3) | (1,4) |
| | N/A | | | |
| target | (2,1) | (2,2) | (2,3) | (2,4) |
| | Repeat | N/A | N/A | |
| reporter | (3,1) | (3,2) | (3,3) | (3,4) |
| | Repeat | Repeat | N/A | |
| coacervate-forming | (4,1) | (4,2) | (4,3) | (4,4) |
| compound | Repeat | Repeat | Repeat | |

| | | | | |
|---|---|---|---|---|
| *potential interactions include: (1,2), (1,3), (1,4), (2,4), (3,4), (4,4) ***other interactions are repeats (mirror across matrix diagonal) or not applicable *****potential functionalization: (1,4), (3,4) | | | | |

### Exemplary reaction formats

1. If the detection moiety comprises a fluorophore (as detection moiety) and a quencher, the cleaved fluorophore is preferentially taken up by coacervates
   a. Interactions: (1,2), (1,3), (3,4), (4,4)
   b. Interactions driven by coacervate: (3,4)
   c. Functionalization (optional): (3,4)
2. Reporter (as a whole) is taken up by coacervate
   a. Interactions: (1,2), (1,3), (3,4), (4,4)
   b. Interactions driven by coacervate: (3,4)
3. Everything (all four reaction components) is concentrated in coacervates
   a. Interactions: (1,2), (1,3), (1,4), (2,4), (3,4), (4,4)
   b. Interactions driven by coacervate: (1,4), (2,4), (3,4)
4. Cas protein (and therefore Cas complex) is bound to a coacervate-forming compound. Functionalized protein interacts with coacervate formed by positively charged coacervate-forming compounds. Coacervate takes up all nucleic acids (target and reporter)
   a. Interactions: (1,2), (1,3), (1,4), (2,4), (3,4), (4,4)
   b. Interactions driven by coacervate: (1,4), (2,4), (3,4)
   c. Functionalization: (1,4)
5. Reporter is attached to a coacervate-forming compound (e.g. with detection moiety (e.g. fluorophore side) of reporter closer to attachment site) and Cas protein is bound to a coacervate-forming compound (as described in 4). Functionalized Cas protein interacts with coacervate formed by positively charged coacervate-forming compounds. Coacervate takes up target, reporter and cleaved detection moiety (e.g. functionalized fluorophore).
   a. Interactions: (1,2), (1,3), (1,4), (2,4), (3,4), (4,4)
   b. Interactions driven by coacervate: (1,4), (2,4), (3,4)
   c. Functionalization: (1,4), (3,4)
6. Cas protein (and therefore Cas complex) is attached to coacervate-forming compound. Functionalized Cas protein interacts with coacervates formed by positively charged coacervate-forming compound. Coacervates do not take up cleaved detection moiety (e.g. fluorophore).
   a. Interactions: (1,2), (1,3), (1,4), (2,4), (4,4)
   b. Interactions driven by coacervate: (1,4), (2,4)
   c. Functionalization: (1,4)
7. Everything (all four reaction components) is concentrated in coacervates, but the cleaved detection moiety (e.g. fluorophore) is not.
   a. Interactions: (1,2), (1,3), (1,4), (2,4), (3,4), (4,4)
   b. Interactions driven by coacervate: (1,4), (2,4), (3,4)
8. Cas protein (and therefore Cas complex) is attached to a coacervate-forming compound. Functionalized protein interacts with coacervates formed by a positively charged coacervate-forming compound (coacervate 1). Reporter is attached to a hydrophobic molecule or multiple dyes. Functionalized uncleaved reporter, functionalized Cas protein and target are taken up in coacervate 1. After cleavage of the nucleic acid sequence of the reporter, the functionalized detection moiety (e.g. fluorophore) is taken up in a hydrophobic coacervate formed by hydrophobic coacervate-forming compounds (coacervate 2). Coacervate 1 and 2 are immiscible (multiphase coacervates).
   a. Interactions: (1,2), (1,3), (1,4), (2,4), (3,4), (4,4)
   b. Interactions driven by coacervate: (1,4), (2,4), (3,4)
   c. Functionalization: (1,4).

In an embodiment of the composition or kit of parts according to the present invention which may be combined with any of the embodiments of the composition or kit of parts preaddressed or still to be addressed unless in contradiction, the composition or kit of parts according to the present invention is one, wherein
(A)
   a. the coacervate-forming compounds are positively charged,
   b. the fluorophore of the reporter is negatively charged, and
   c. the Cas protein is bound, optionally covalently bound, to a coacervate-forming compound; or
(B)
   a. the coacervate-forming compounds are neutral in charge,
   b. the Cas protein is not bound to a coacervate-forming compound, and
   c. the fluorophore of the reporter is bound, optionally covalently bound, to a coacervate-forming compound; or
(C)
   a. the coacervate-forming compounds are hydrophobic,
   b. the Cas protein is not bound to a coacervate-forming compound, and
   c. the reporter is bound, optionally covalently bound, to the fluorophore and comprises a hydrophobic molecule, optionally cholesterol, digoxigenin, biotin; or
(D)
   a. the coacervate-forming compounds are positively charged,
   b. the Cas protein is bound, optionally covalently bound, to a coacervate-forming compound or a negatively charged polymer; or
(E)
   a. the coacervate-forming compounds are selected from positively charged compounds and from hydrophobic compounds,
   b. the coacervate-forming compounds form two immiscible phases,
   c. the Cas protein is bound, optionally covalently bound, to a coacervate-forming compound or a negatively charged polymer; or
   d. the reporter is bound, optionally covalently bound, to the fluorophore and comprises a hydrophobic molecule.

Further objectives are achieved by a use and method according to the appended claims.

In an aspect which may be combined with any of the embodiments or aspects preaddressed or still to be addressed unless in contradiction, the present invention is directed to a use of the composition or kit of parts as described herein for:
a. the detection and/or quantification of nucleic acids,
b. the detection of environmental DNA for monitoring and surveillance,
c. the detection of weeds, invasive species, plant viruses and/or plant virus infections,
d. the detection of DNA for personal identification,
e. the diagnosis of a disease, optionally an infection or a mutation,
f. the detection of antimicrobial resistances, and/or
g. the detection of environmental pollutants.

In the context of the use, the detection and/or quantification of nucleic acids refers to the nucleic acids which are the target suitable for activating the Cas complex.

In an aspect which may be combined with any of the embodiments or aspects preaddressed or still to be addressed unless in contradiction, the present invention is directed to a method for the detection and/or quantification of nucleic acids comprising the steps:
(i) providing and mixing a composition as defined herein in an aqueous medium to form coacervates from the coacervate-forming compounds,
(ii) providing and mixing a nucleic acid target, optionally a cell lysate; a biofluid, optionally blood, saliva, urine, mucus, semen, vaginal fluid, feces, cerebrospinal fluid, amniotic fluid, bone marrow, milk, plasma, tears, sputum, serum, gastrointestinal fluid, or peritoneal fluid; Buccal, nasal, or vaginal swabs; wastewater, saltwater, freshwaters, soil, or soil extract; plant cells, hair, skin cells or microbiome cells with the composition of (i),
(iii) detecting and/or quantifying the nucleic acid target based on the signal of the detection moiety, optionally the fluorescent detection moiety.

In the context of the present method, the nucleic acid of step (ii) can, for example, be comprised in a cell lysate; a biofluid, optionally blood, saliva, urine, mucus, semen, vaginal fluid, feces, cerebrospinal fluid, amniotic fluid, bone marrow, milk, plasma, tears, sputum, serum, gastrointestinal fluid, or peritoneal fluid; Buccal, nasal, or vaginal swabs; wastewater, freshwaters, soil, or soil extract. Alternatively, the nucleic acid of step (ii) can be isolated, e.g. from one of the sources mentioned in the foregoing sentence, for use in this step.

In an embodiment, the method according to the present invention is one, wherein the method is for the diagnosis of a disease, optionally an infection or a mutation, antimicrobial resistances, biological and/or environmental pollutants.

The following Figures and Examples serve to illustrate the invention and are not intended to limit the scope of the invention as described in the appended claims.

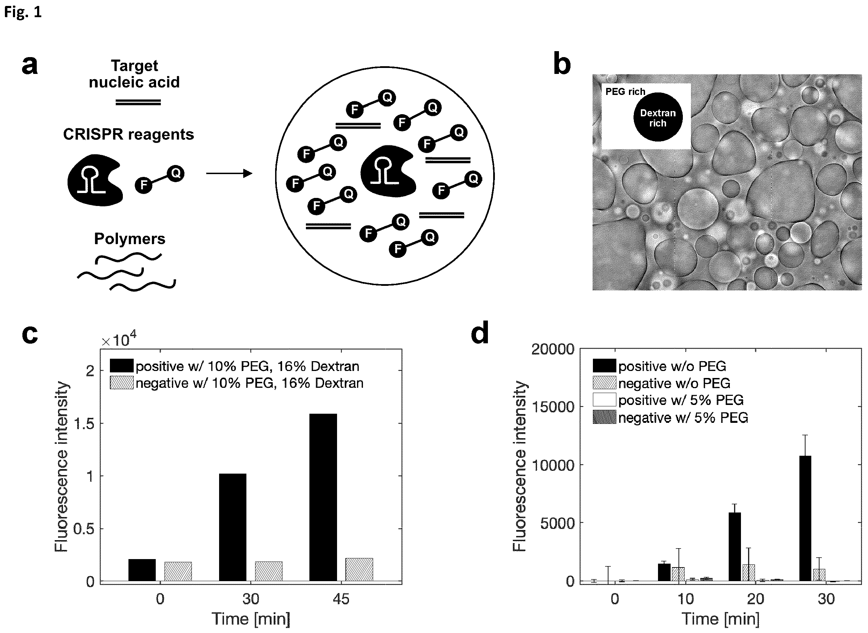

Regarding suitable coacervate-forming compounds and coacervates, reference is made to Figs. 1 to 7 of WO2022/122675 (incorporated by reference).
**Figure 1** shows CRISPR reaction in an aqueous two phase system. **a)** depicts a schematic illustration of the strategy of the invention. **b)** Formation of dextran-rich compartments (coacervates) in PEG-rich phase (V_{dextran}:V_{PEG} 1:10). The two phases were obtained from aqueous two phase system (ATPS) of 10 wt/wt % PEG/ 16% wt/wt dextran prepared in 1x NEB 2.1 buffer. **c)** Fluorescence intensity measurements of a CRISPR Cas reaction run in the ATPS described in (b). The reaction successfully ran in an ATPS system. The negative sample did not contain target DNA, whereas the positive sample did. **d)** The CRISPR reaction in 1x NEB 2.1 supplemented with 5% PEG did not run. As a result, the reaction worked in an ATPS system with a higher PEG concentration because of compartmentalization of reagents in the dextran-rich phase.

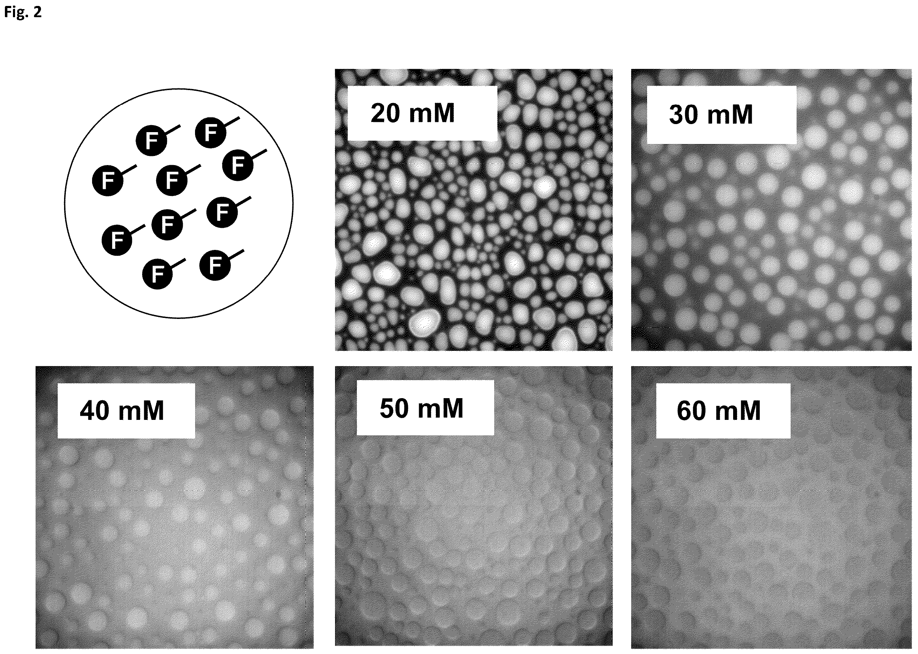

**Figure 2** shows microscopy images of unquenched fluorophore in positively-charged compartments formed by coacervate-forming compounds. The strength of the interaction of the DNA with the compartments could be controlled with the amount of unpaired charges, i.e. by tuning the salt concentration or the polymer composition.

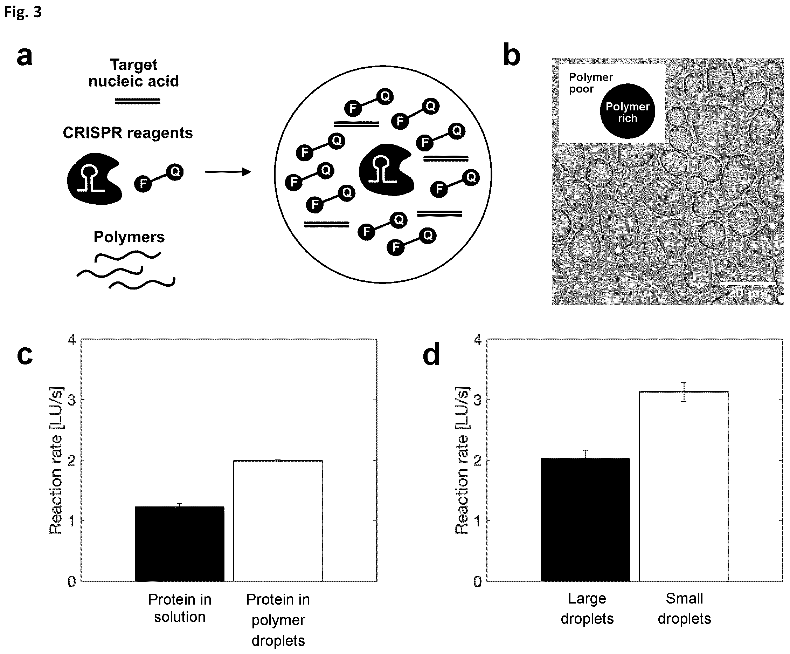

**Figure 3** shows CRISPR reaction in positively-charged coacervates. **a)** depicts a schematic illustration of the strategy of the invention. **b)** Image of positively-charged compartments formed by mixing a positively-charged coacervate-forming compound (0.5 mg mL⁻¹) in 1x HOLMES buffer. **c)** The rate of reaction observed for a system with functionalized Cas and positively-charged coacervates was significantly greater than that observed for unfunctionalized Cas without coacervates in the same reaction conditions and after the same time. Thus, the invention positively impacts the state-of-the-art CRISPR reaction. **d)** The rate of reaction of a system with functionalized Cas and positively-charged coacervates can be further improved, e.g. changing the size of the positively charged droplets.

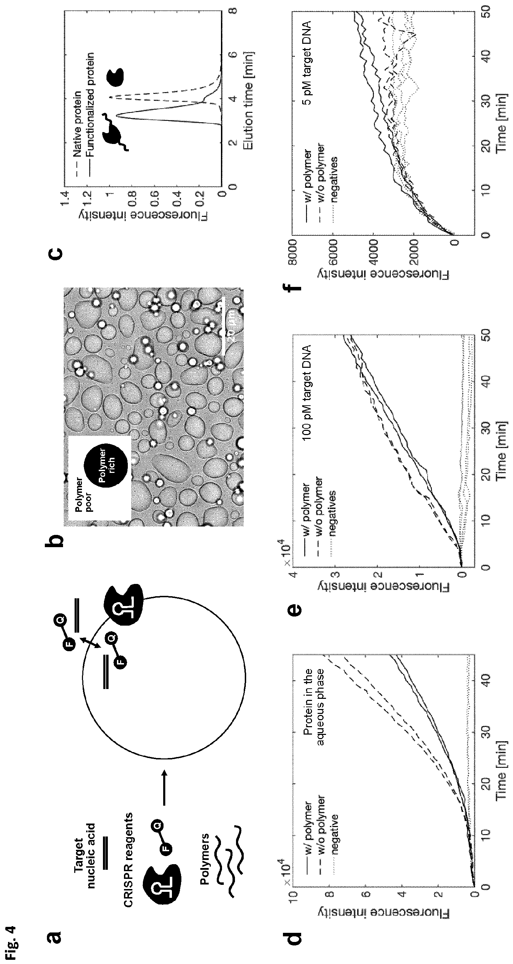

**Figure 4** shows CRISPR reaction at the interface of positively-charged coacervates. **a)** depicts a schematic illustration of the strategy of the invention. **b)** Image of positively-charged compartments formed by mixing a positively-charged coacervate-forming compound (0.5 mg mL⁻¹) in 1x HOLMES buffer. **c)** Size exclusion chromatography analysis of the functionalized protein. A Cas protein functionalized with neutral coacervate-forming compounds was used. **d)** The CRISPR reaction with the functionalized protein occurred at a lower rate when the protein was first incubated with the coacervates and the coacervates were removed before the reaction was started (i.e. with the addition of the reporter and the target). Thus, the functionalized protein was partially taken up inside the coacervates. **e)** The CRISPR reaction with the functionalized protein proceeded at a similar rate with and without positively-charged coacervates in solution. Thus, the protein taken up in the coacervates was mostly exposed to the reaction mixture. **f)** When the target concentration was low (e.g. 5 pM), the positively-charged coacervates promoted interaction between the protein and the target and yielded a signal, otherwise undetectable. In this system, the nucleic acids in solution only weakly interacted with the positively-charged coacervates.

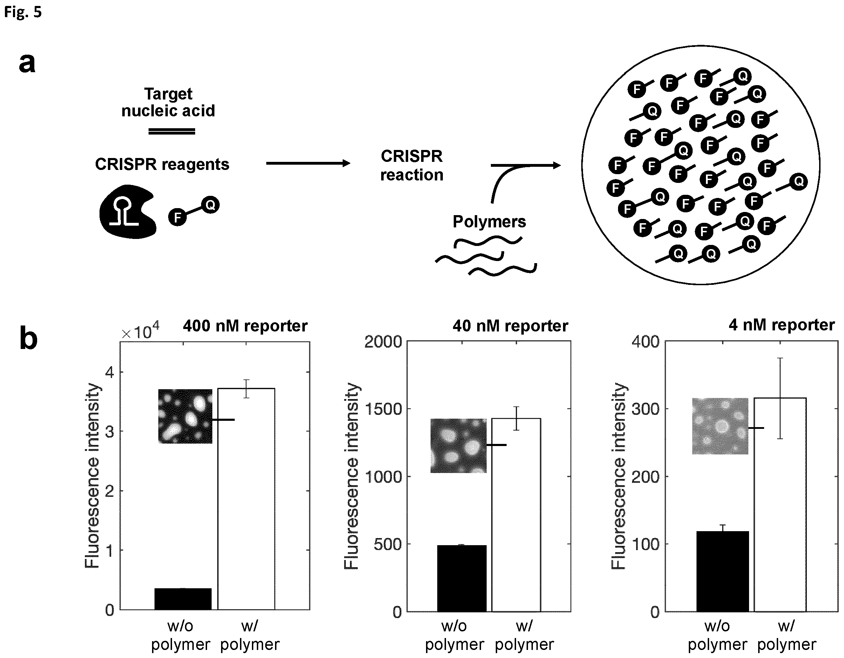

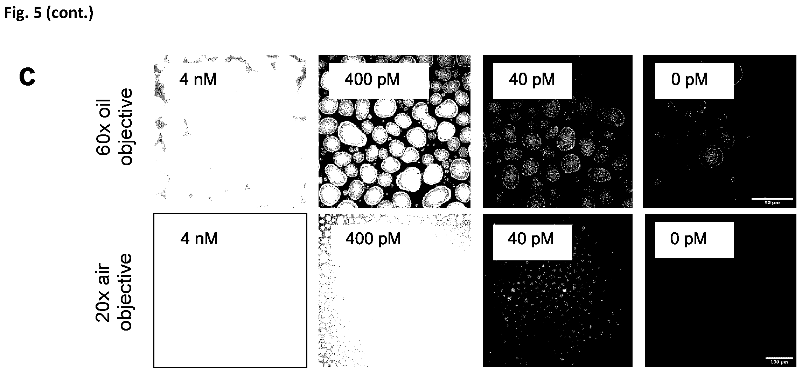

**Figure 5** shows recruitment of cleaved fluorophore in liquid droplets. **a)** depicts a schematic illustration of the strategy of the invention. **b)** Fluorescence intensities of samples with and without the coacervate-forming compounds. Focusing unquenched fluorophore in the coacervates helped to significantly increase the fluorescence intensity of the sample, thus simplifying detection, **c)** The reporter cleaved during the CRISPR reaction could be recruited in positively-charged compartments after the reaction. The fluorescence signal in the droplets could be detected by microscopy at both higher and lower magnification.

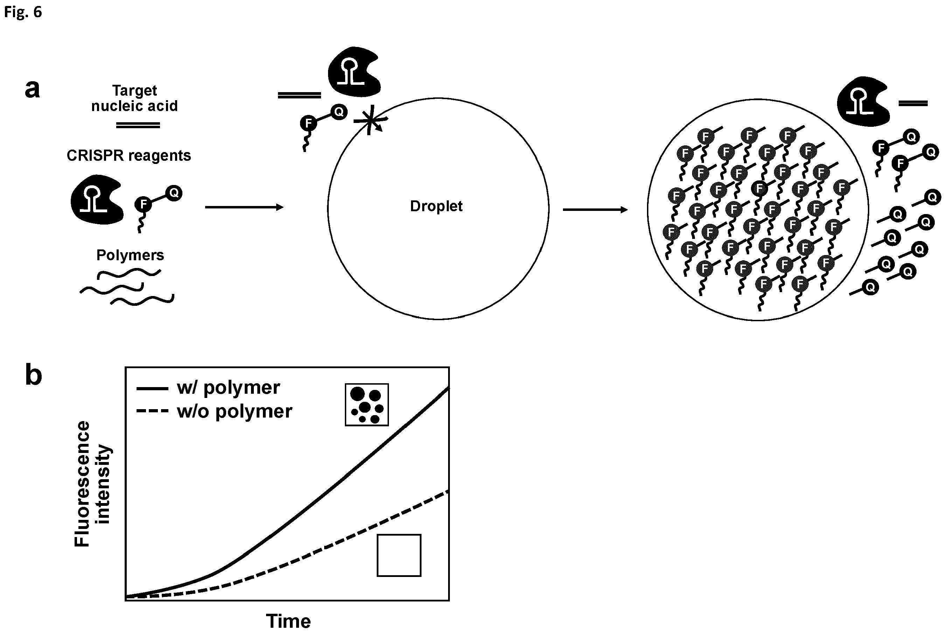

**Figure 6** shows recruitment of cleaved fluorophore in liquid droplets during the CRISPR reaction. **a)** depicts a schematic illustration of the strategy of the invention. The CRISPR reaction occurred in solution. Upon cleavage of the reporter, the fluorophore side of the reported was taken up in the liquid droplets. As an example, the fluorophore side could be functionalized with a hydrophobic moiety, and was recruited in a hydrophobic coacervate only upon cleavage. **b)** This setup led to the uptake of fluorophore in time and facilitated result detection.

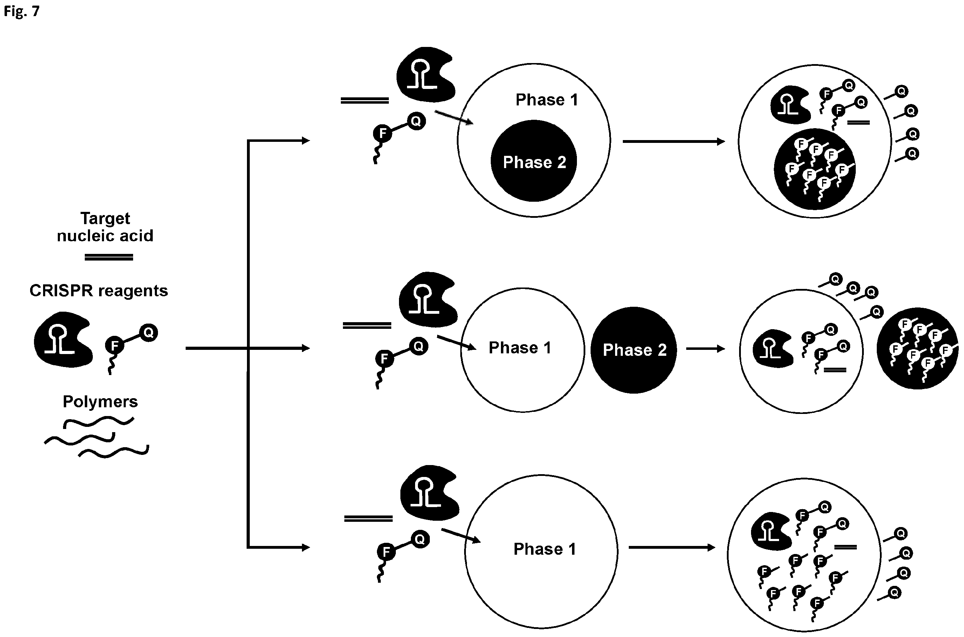

**Figure 7** shows coacervates used to focus the reaction and the fluorescent signal at once. Multiple coacervate-forming compounds were added to form two immiscible phases (row 1 and 2), where one focused the CRISPR reaction and the other focused the cleaved fluorophore. Alternatively, a coacervate-forming compound was added to focus the reaction and the cleaved fluorophore in the same compartment (row 3).

### Examples

Regarding suitable coacervate-forming compounds and coacervates, reference is made to Examples 1 to 23 of WO2022/122675 (incorporated by reference).

### Example 1

A Cas complex was prepared with LbCas12a and gRNA as described in literature (J. Chen et al., Science 2018, 360, 6387, 436-439). The gRNA was designed to detect the gene for RNAseP. An aqueous two phase system (ATPS) of 10 wt/wt % PEG/ 16% wt/wt dextran was prepared in 1x NEB 2.1 buffer. PEG 8kDa (Sigma Aldrich) and dextran 40kDa (Sigma Aldrich) were used. After centrifugation, the PEG-rich and the dextran-rich phase were separated into two clear phases. The two phases were then separately mixed at a 1:10 V_{dextran/}V_{PEG} ratio with the Cas complex, the reporter (FAM-TTATTATT-BHQ1 (SEQ ID NO: 45)) and the target respectively, and incubated for 2h at room temperature. After 2h the three solutions were mixed and incubated at 37°C to start the reaction. The amounts of the Cas complex, the reporter and the target were selected to achieve final concentrations of 27 nM, 400 nM and 100 pM, respectively. In the negative sample, the target was not added. At discrete time intervals, aliquots of the reaction mixture were taken and their fluorescence measured using a plate reader.

### Example 2

A Cas complex was prepared with Cas14 and gRNA as described in literature (L.B. Harrington et al., Science 2018, 362, 6416, 839-842). The gRNA was designed to detect the gene for HERC2 gene. An aqueous two phase system (ATPS) of 10 wt/wt % PEG/ 16% wt/wt dextran was prepared in 1x NEB 2.1 buffer. PEG 8kDa (Sigma Aldrich) and dextran 40kDa (Sigma Aldrich) were used. After centrifugation, the PEG-rich and the dextran-rich phase were separated into two clear phases. The two phases are then separately mixed at a 1:10 V_{dextran/}V_{PEG} ratio with the Cas complex, the reporter (FAM-TTTTTTTTTTTT-BHQ1 (SEQ ID NO: 46)) and the target respectively, and incubated for 2h at room temperature. After 2h the three solutions were mixed and incubated at 37°C to start the reaction. The amounts of the Cas complex, the reporter and the target were selected to achieve final concentrations of 100 nM, 400 nM and 100 pM, respectively. In the negative sample, the target was not added.

### Example 3

LbCas12a is covalently conjugated to negatively charged polymers with the following structure 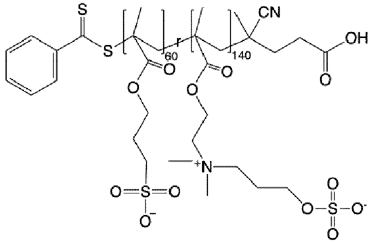 using a protocol previously described (in WO2022/122675). "r" means random. The conjugation and protein concentration were verified and measured using size exclusion chromatography. A coacervate-forming compound with the following structure 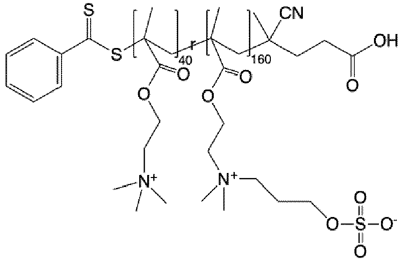 was used. "r" means random. The Cas conjugate, the gRNA against RNAseP, the reporter (FAM-TTATTATT-BHQ1 (SEQ ID NO: 45)), the RNAseP target and the coacervate-forming compound were mixed in 1x HOLMES buffer to achieve final concentrations of 27 nM, 27 nM, 400 nM, 100 pM and 0.5 mg mL⁻¹, respectively. The final NaCl concentration was 40 mM. The reaction was run at 37°C and the fluorescence measured using a plate reader. In this setup, the Cas conjugate and the target were mostly inside the positively-charged compartments, while the reporter weakly interacted with them.

### Example 4

LbCas12a was covalently conjugated to negatively charged polymers with the following structure 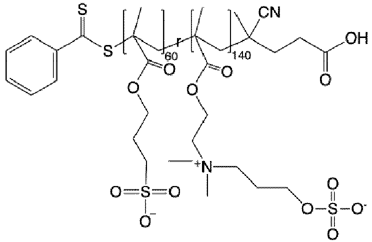 using a protocol previously described (in WO2022/122675). "r" means random. The conjugation and protein concentration were verified and measured using size exclusion chromatography. A coacervate-forming compound with the following structure 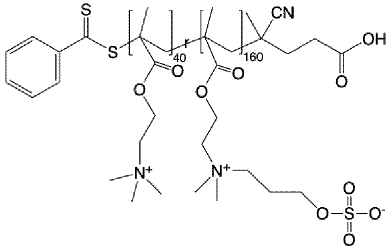 was used. "r" means random. The Cas conjugate, the gRNA against HPV16, the reporter (FAM-TTATTATT-BHQ1 (SEQ ID NO: 45)), the HPV16 target and the coacervate-forming compound were mixed in 1x HOLMES buffer to achieve final concentrations of 27 nM, 27 nM, 400 nM, 100 pM and 0.125 mg mL⁻¹, respectively. The final NaCl concentration is 40 mM. The reaction was run at 37°C and the fluorescence measured using a plate reader.

### Example 5

LbCas12a was covalently conjugated to zwitterionic polymers with the following structure 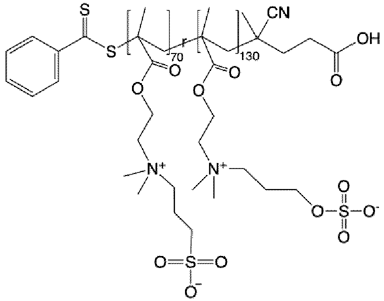 using a protocol previously described (in WO2022/122675). "r" means random. The conjugation and protein concentration were verified and measured using size exclusion chromatography. A coacervate-forming compound with the following structure 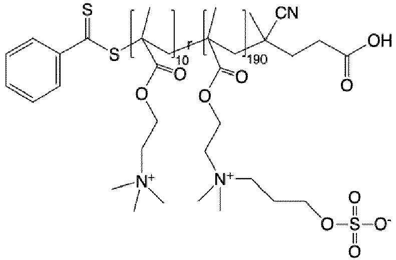 was used. "r" means random. The Cas conjugate, the gRNA against RNAseP, the reporter (FAM-TTTTTTTTTTTT -BHQ1 (SEQ ID NO: 46)), and the coacervate-forming compound were mixed in 1x HOLMES buffer to achieve final concentrations of 27 nM, 27 nM, 400 nM, and 0.5 mg mL⁻¹, respectively. The RNAseP target was added to achieve final concentrations of either 100 pM or 5 pM. The final NaCl concentration was 26 mM. The reaction was run at 37°C and the fluorescence measured using a plate reader. In this setup, the Cas conjugate was partially on the surface of the positively-charged compartments, at the interface between the two immiscible phases, while the reporter and the target weakly interacted with them.

### Example 6

Unquenched reporter (FAM-TTATTATT (SEQ ID NO: 47)) was mixed with coacervate-forming compounds with the following structure 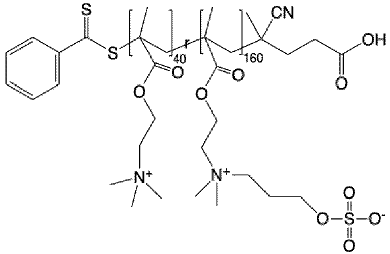 in 1x NEB 2.1 buffer. "r" means random. The final concentrations of reporter were 400 nM, 40 nM and 4 nM, of coacervate-forming compound 0.1 mg mL⁻¹ and of NaCl 18 mM. Microscopy images of wells with and without the coacervate-forming compounds were taken after an incubation of 1-5 min using a laser power of 3% and an exposure time of 500 ms in all cases. The fluorescence intensity of the solutions and the droplets were analyzed using an image processing software.

### Example 7

A Cas complex was prepared with LbCas12a and gRNA as described in literature (J. Chen et al., Science 2018, 360, 6387, 436-439). The gRNA was designed to detect the gene for RNAseP. The Cas complex and the reporter (FAM-TTATTATT-BHQ1 (SEQ ID NO: 45)) were mixed in 1x NEB 2.1 buffer to achieve final concentrations of 27 nM and 400 nM, respectively. The RNAse P target was added to achieve final concentrations of 4 nM, 400 pM, 40 pM, 4 pM, 400 fM and 40 fM in the positives, and was not added in the negatives. The solutions were first incubated at 37°C for 20 min, then diluted 5x with Nuclease free water and mixed with a coacervate-forming compound (final concentration 0.25 mg mL⁻¹) with the following structure 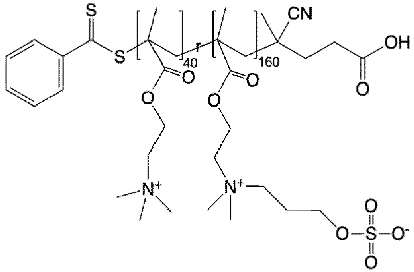 "r" means random. The droplets were imaged after 5 min by microscopy using a 20x air objective.

## Claims

1. A composition or kit of parts comprising:
(1) coacervate-forming compounds,
(2) a Cas complex comprising a guide RNA and a Cas protein, wherein the Cas protein has trans or collateral cleavage activity for a nucleic acid sequence of a reporter,
(3) a reporter comprising a detection moiety covalently linked to the nucleic acid sequence, wherein the nucleic acid sequence is suitable for being cleaved by the Cas complex, and the detection moiety is suitable for optical detection, optionally fluorescence, colorimetric, or luminescence detection, or electrochemical detection; and
(4) optionally a target suitable for activating the Cas complex,
wherein
at least one, optionally at least two or three, of the coacervate-forming compounds, the Cas complex, the reporter, the cleaved detection moiety and/or the target are configured such that at least one, optionally at least two or three, of the Cas complex, the reporter, the cleaved detection moiety and/or the target are taken up by coacervates formed by the coacervate-forming compounds.

2. The composition or kit of parts according to claim 1, wherein
(A) the detection moiety is selected from the group consisting of: one or more fluorophore(s) covalently bound via the nucleic acid to one or more quenchers; metal nanoparticles, optionally gold nanoparticles; a molecule suitable for colorimetric analysis, optionally horseradish peroxidase, calf intestine alkaline phosphatase, 3,3',5,5'-tetramethylbenzidine (TMB), 2,2'-azinobis [3-ethylbenzothiazoline-6-sulfonic acid]-diammonium salt (ABTS), *o*-phenylenediamine dihydrochloride (OPD), *p*-nitrophenyl Phosphate (*p*-NPP), luminol, polyphenols, acridine esters, and luciferin; and a redox-active moiety suitable for electrochemical detection;
(B) the detection moiety is one or more fluorophore(s) covalently linked to the nucleic acid sequence and one or more quencher(s) for the fluorophore(s), wherein the nucleic acid is suitable for being cleaved by the Cas complex to separate the fluorophore(s) and the quencher(s); and/or
(C) the nucleic acid sequence is a ssDNA, ssRNA, dsDNA, dsRNA, DNA hairpin, a locked nucleic acid (LNA) or peptide nucleic acid (PNA), wherein the nucleic acid optionally comprises or consists of artificial nucleic acid base pair analogs.

3. The composition or kit of parts according to claim 1 or 2, wherein the coacervate-forming compounds comprise or consist of
a. a self-associating polymer,
b. cationic and anionic molecules, optionally mixed in ratios so that a coacervate including at least the Cas complex, the target, the reporter or the cleaved detection moiety is formed, optionally in ratios between 0.2 mol/mol to 0.8 mol/mol cationic to anionic, optionally polycations, polyanions, polyU, proteins, peptides, nucleic acids, small molecules, optionally ATP, ADP, counterions, poly-lysine, poly-arginine, poly-aspargine, polyethylenimine, polyamidoamine, mannosylated polyethylenimine, or chitosan, and
c. polyethylene glycol, dextran, sucrose, polysucrose, glycerol, sorbitol, copolymerized sucrose and epichlorohydrin, Ficoll, poly(2-ethyl-2-oxazoline), poly(vinyl alcohol), glucose, bovine serum albumin, ionic liquids, double hydrophilic block copolymers, optionally pullulan-b-poly(N,N-dimethylacrylamide), poly(ethylene glycol)-b-poly(2-methyl-2-oxazoline), poly(ethylene glycol)-b-dextran, polyethylene glycol)-b-pullulan, dextran-b-poly(sarcosine)), or a combination of these, optionally a combination with alcohols, salts and/or amino acids, optionally ethanol, 1-propanol, 2-propanol, phosphates, sulfates or citrates.

4. The composition or kit of parts according to claim 3, wherein the self-associating polymer is selected from the group consisting of:
(A) a polymer exhibiting phase transition when the temperature is decrease below the critical solution temperature of the polymer, optionally a zwitterionic polymer, ureido polymers and optionally derivatives thereof, optionally poly(allylamine)-co-poly(allylurea), poly(allylurea), poly(ureidoethylmethacrylate), poly(citrulline), poly(allylamine)-co-poly(allylurea) and optionally derivatives thereof, poly(N-acryloylglycinamide) and optionally derivatives thereof, poly(*N*-acryloylasparaginamide) and optionally derivatives thereof, poly(*N*-(2-hydroxypropyl) methacrylamide)-glycolamide) and optionally derivatives thereof, or copolymers of acrylamide and acrylonitrile,
(B) a polymer exhibiting phase transition when the temperature is increased beyond the critical solution temperature of the polymer, optionally poly(N-isopropyl acrylamide) (PNIPAM), poly(2-(N-(dimethylamino) ethyl methacrylate) (PDMAEMA), poly(ethylene glycol) methacrylate (PEGMA), poly(N-vinyl caprolactam) (PNVCL), poly(phenylene oxide) (PPO), poly(2-oxazolines) (Pox), poly(N,N-diethyl acrylamide), poly(oligoethylene glycol [methyl ether] [meth]acrylates), poly(methyl vinyl ether), polypropylene glycol), and
(C) a protein or peptide exhibiting phase transition, optionally
a. an elastin-like polypeptide comprising or consisting of at least two repeats having the amino acid formula (V-P-G-X-G)ₙ (SEQ ID NO: 40), wherein X is any natural amino acid except proline and n is an integer from 2 to 600, or
b. a low complexity domain (LCD) stretch of naturally occurring nucleic acid binding proteins,
- optionally of transcription factors or RNA binding proteins, optionally selected from the group consisting of Dhh1, Dbp1, Laf1, Ddx4, hNRNPA1, FUS, EWS and hnRNPNA2,
- optionally proteins comprising or consisting of an amino acid sequence having at least 85%, 87.5%, 90%, 92%, 94%, 95%, 96%, 97%, 98%, or 99% sequence identity and/or amino-acid composition identity to Dhh1, Dbp1, Laf1, Ddx4, hNRNPA1, FUS, EWS or hnRNPNA2, optionally over the whole sequence, and
- proteins in which not more than 5, optionally not more than 3, 2 or 1 amino acid(s) is (are) removed from the sequence of the RNA binding proteins selected from the group consisting of Dhh1, Dbp1, Laf1, Ddx4, hNRNPA1, FUS, EWS and hnRNPNA2 at one or both ends thereof,
c. a resilin-like polypeptide comprising or consisting of at least two identical or different repeats having the amino acid formula (GRGDSPYS)ₙ (SEQ ID NO: 41) or (GGRPSDSYGAPGGGN)ₙ (SEQ ID NO: 42) or (GYSGGRPGGQDLG)ₙ (SEQ ID NO: 43)or (AQTPSSQYGAP)ₙ (SEQ ID NO: 44), wherein n is an integer from 2 to 600, optionally a resilin-like polypeptide comprising or consisting of an amino acid sequence having at least 50%, optionally at least 75% or 90%, sequence identity and/or amino-acid composition identity to the resilin-like polypeptide comprising or consisting of at least two identical or different repeats having the amino acid formula (GRGDSPYS)ₙ or (GGRPSDSYGAPGGGN)ₙ or (GYSGGRPGGQDLG)ₙ or (AQTPSSQYGAP)ₙ, wherein n is an integer from 2 to 600.

5. The composition or kit of parts according to claims 3 or 4, wherein the self-associating polymer, optionally zwitterionic polymer, is according to Formula (I): 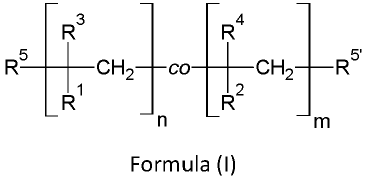 wherein
**n** is an integer selected from 0 to 2500, optionally from 25 to 250;
**m** is an integer selected from 2 to 2500, optionally from 25 to 250;
**R³** and **R⁴** are each independently selected from the group consisting of hydrogen and methyl;
**R⁵** and **R^{5'}** are independently selected from
- hydrogen, linear or branched (C₁₋₁₀)alkyl, a halide and phenyl, or
- a group resulting from a polymerization initiator, optionally a group resulting from a RAFT polymerization initiator, optionally 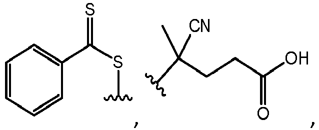 or a target binding moiety (TBM), optionally a TBM attached to or reacted with a group resulting from a polymerization initiator, optionally from a RAFT polymerization initiator, optionally 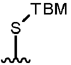 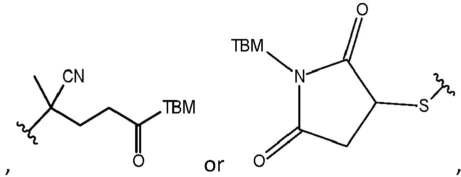 wherein the TBM is optionally selected from the group consisting of azide, alkyne, carboxylate, amine, thiol, hydroxyl, aldehyde, cyanate, sulfonyl, tosyl, tresyl, epoxide, carbonate, anhydride, carbamate, imidazole, azlactone, triazine, maleimide, aziridine, peroxide, acyl, anthraquinone, diazo, diazirine, psoralen, NHS ester, imido ester, (C₂₋₁₀)alkenyl or alkyl groups, DBCO, cytosine, guanidine, streptavidin, avidin, biotin, an aptamer, a peptide, optionally a peptide comprising an affinity tag, optionally an affinity tag selected from FLAG, HIS and ALFA, a protein, an oligonucleotide, a positively or negatively charged oligopolymer, a molecularly imprinted polymer, an affinity polymer, a nucleic acid, a carbohydrate, a dye, a cell, an antibody, an organic and inorganic nanoparticle, and chelating agents, optionally ethylenediaminetetraacetic acid (EDTA);
**R**¹ is selected from the group consisting of
(i) -X-hydrogen, -X-methyl, -X-ethyl and -X-propyl;
(ii) 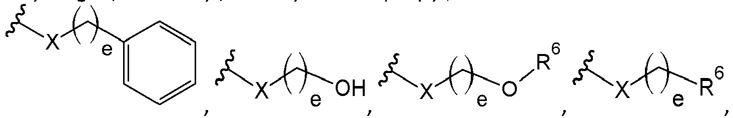 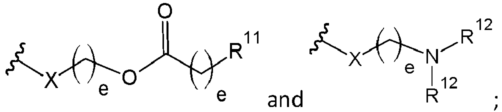
(iii) 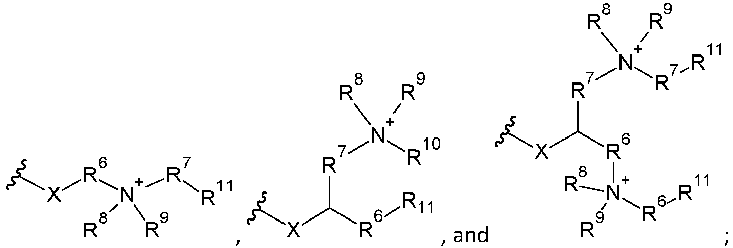
(iv) 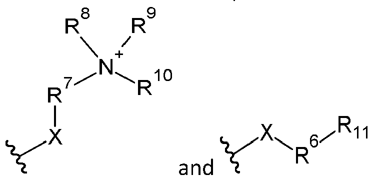 in a ratio of about 1:1 within the polymer;
(v) 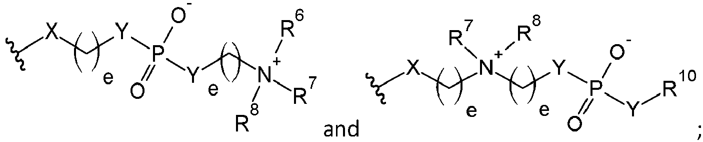 and
(vi) 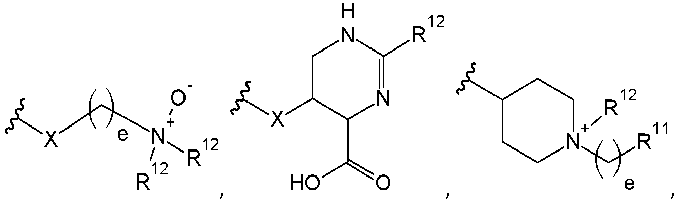 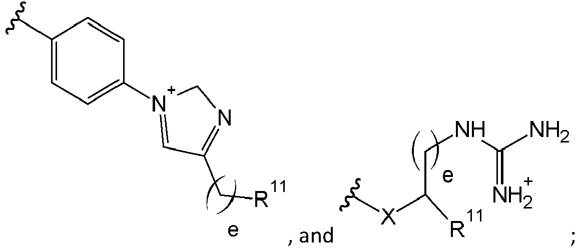
**R²** is selected from the group consisting of 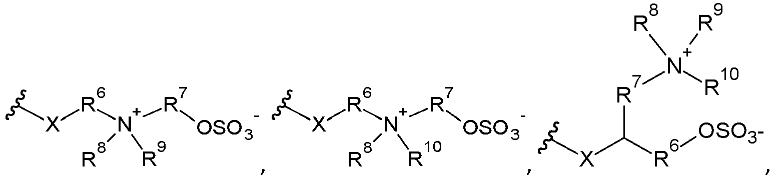 and 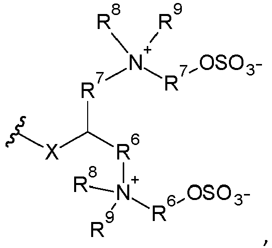 wherein
**X** is selected from the group consisting of -C(=O)-O-, -C(=O)-NH-, aliphatic and aromatic carbo- or heterocycle;
**Y** is oxygen or absent;
**e** is an integer selected from 1 to 5, optionally from 2 to 3;
**R⁶** and **R⁷** are each independently selected from the group consisting of
linear or branched, substituted or non-substituted (C₁₋₁₀)alkyl, optionally (C₁₋₅)alkyl, optionally methyl, ethyl and propyl,
linear or branched, substituted or non-substituted (C₁₋₁₀)alkyl, optionally (C₂₋₆)alkyl chains, comprising one or more heteroatoms selected from the group consisting of O, N, S and P, optionally (C₂₋₅)alkyl ethers,
(C₂₋₁₀)alkenyl,
phenyl, and an aromatic heterocycle, optionally pyridine, pyrrole, furan and imidazole;
**R⁸, R⁹** and **R¹⁰** are each independently selected from the group consisting of hydrogen, linear or branched, substituted or non-substituted (C₁₋₁₀)alkyl, optionally (C₁₋₅)alkyl, optionally methyl, ethyl and propyl, (C₂₋₁₀)alkenyl, phenyl, an aromatic heterocycle, optionally pyridine, pyrrole, furan and imidazole, 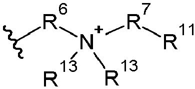 when forming R¹, 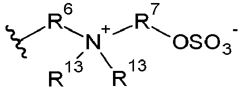 when forming R²;
**R**⁸ and **R⁹** together or two **R¹³** together optionally form an aliphatic or aromatic heterocycle, optionally a piperidine ring, a piperazine ring or a morpholine ring;
**R⁸, R⁹** and **R⁶ or R⁸, R⁹** and **R⁷** optionally together form a five or six-membered ring, optionally a pyridine ring, a piperidine ring, pyrrole ring, a pyrimidine ring, a pyrazole ring, an imidazole ring, a pyrazine ring, an isoxazole ring or an oxazole ring; wherein one of **R⁸ or R⁹** is absent if **R⁸** and **R⁶, R⁹** and **R⁶** or **R⁸** and **R⁷,** or **R⁹** and **R⁷** together form an aromatic ring;
**R¹¹** is selected from the group consisting of -SO₃⁻, -C(=O)O⁻, and 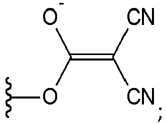
**R¹²** is selected from the group consisting of methyl, ethyl and propyl;
**R¹³** is independently selected from the group consisting of linear or branched, substituted or non-substituted (C₁₋₁₀)alkyl, optionally (C₁₋₅)alkyl, optionally methyl, ethyl and propyl, (C₂₋₁₀)alkenyl, phenyl, an aromatic heterocycle, optionally pyridine, pyrrole, furan and imidazole,
wherein the polymer optionally is crosslinked by a crosslinker, optionally a crosslinker as R¹.

6. The composition or kit of parts according to claim 5, wherein
**R¹** is selected from the group consisting of 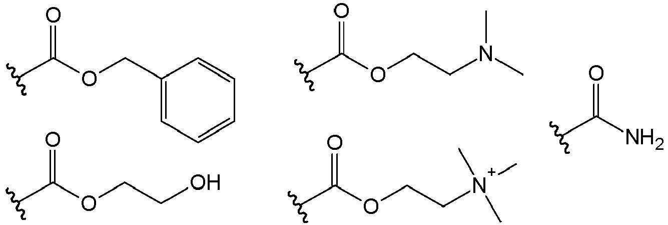 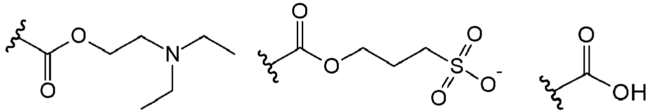 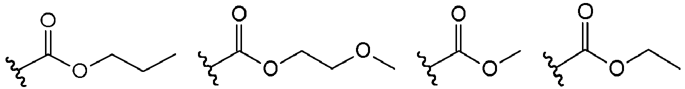 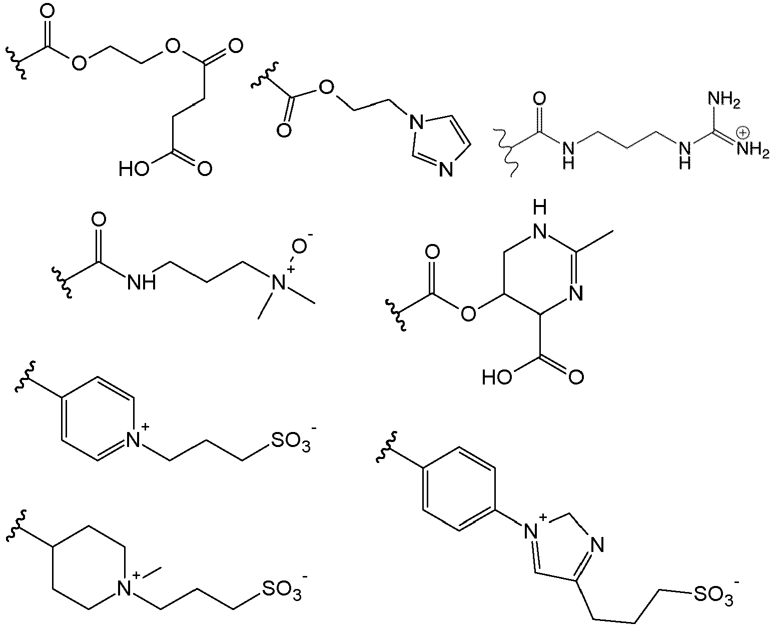 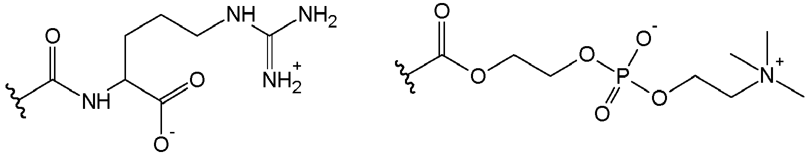 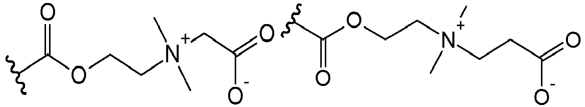 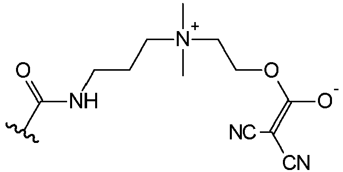 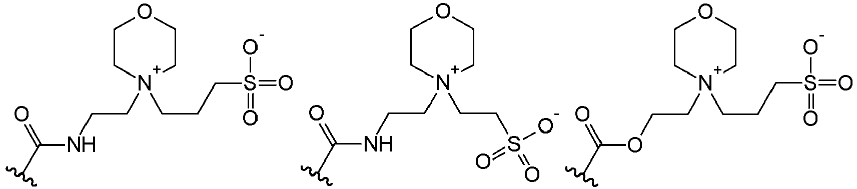 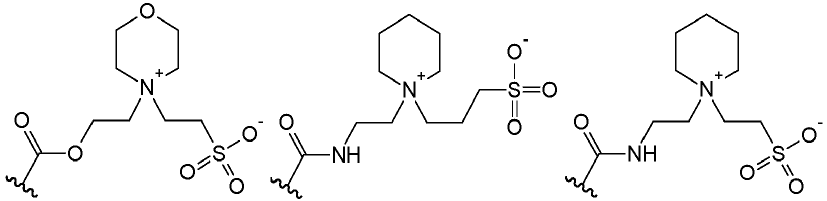 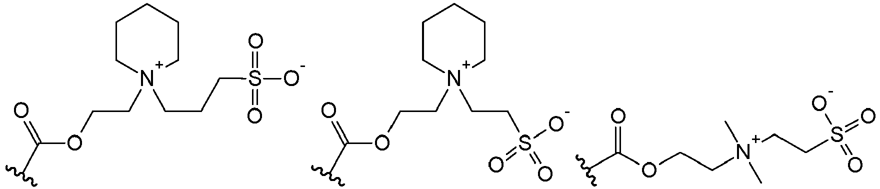 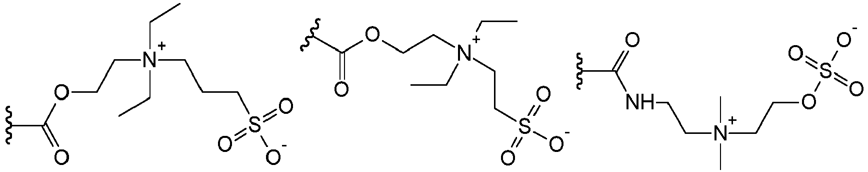 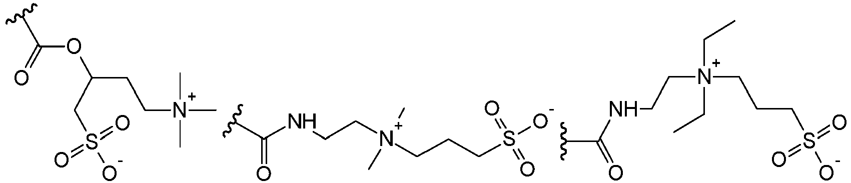 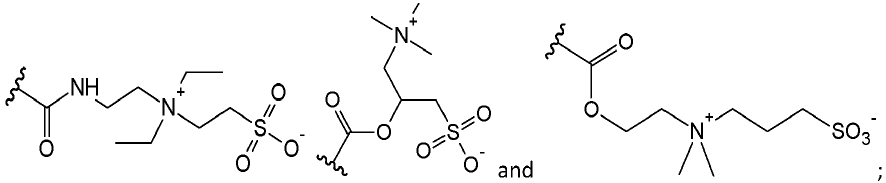 and
**R²** is

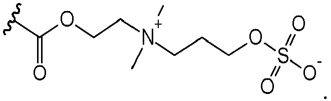

7. The composition or kit of parts according to any of claims 1 to 6, wherein the Cas protein is selected from type I Cas, optionally Cas 3; type II Cas, optionally Cas 9; type III Cas, optionally Cas 10; type V Cas, optionally Cas 12 and Cas14; and type VI Cas, optionally Cas 13.

8. The composition or kit of parts according to any of claims 1 to 7, wherein the Cas protein
a. is bound to a coacervate-forming compound as defined in the preceding claims,
b. is bound, optionally covalently bound, at the N-terminus, C-terminus or both termini to an elastin-like polypeptide as defined in the preceding claims,
c. is bound, optionally covalently bound, at the N-terminus, C-terminus or both termini to a low complexity domain (LCD) stretch from naturally occurring nucleic acid binding proteins as defined in the preceding claims,
d. is bound, optionally covalently bound, at the N-terminus, C-terminus or both termini to a resilin-like polypeptide as defined in the preceding claims,
e. has a TBM at least at the N-terminus, the C-terminus or both termini, optionally selected from the group consisting of
(i) an amine group of the N-terminus or a carboxylate group of the C-terminus,
(ii) a sulfhydryl group of cysteine, a guanidino group of arginine, a phenolate ring of tyrosine, an indole ring of tryptophan, a thioether ring of methionine, and an imidazole ring of histidine,
(iii) an azide, DBCO, alkyne, alkene and acetyl group, optionally an azide or an alkyne group of a non-canonical amino acid,
(iv) an affinity tag, optionally an alkaline phosphatase, AU1 epitope, AU5 epitope, T7-tag, V5-tag, biotin-carboxy carrier protein, B-tag, calmodulin binding peptide, chloroamphenicol acetyl transferase, cellulose binding domain, chitin binding domain, choline-binding domain, DHFR, E2 epitope, FLAG epitope, galactose binding protein, EE-tag, Glutathione S-transferase, Human influenza hemagglutinin, HaloTag, Histidine affinity tag, HSV epitope, Maltose-binding protein, Myc epitope, PDZ domain, polyhistidine, polyarginine, polyaspartic acid, polycystein, polyphenalanine, S-tag, streptavidin binding peptide, staphylococcal protein A, staphylococcal protein G, Strep-tag, Tandem affinity purification (TAP), T7 epitope, TrpE, and VSV-G, and
(v) avidin, biotin, an aptamer, an oligonucleotide, a molecularly imprinted polymer, an affinity polymer, a carbohydrate, a dye, an organic or inorganic nanoparticle, and a positively or negatively charged polymer.

9. The composition or kit of parts according to any of claims 1 to 8, wherein the reporter comprises a TBM, optionally an azide, an alkyne, a DBCO, a maleimide, a NHS ester, an epoxide, a biotin, a hydrophilic molecule, a charged molecule, a hydrophobic molecule, optionally cholesterol, digoxigenin, biotin, or a coacervate-forming compound as defined in any of the preceding claims.

10. The composition or kit of parts according to any of claims 1 to 9, wherein
A. the coacervate-forming compounds, the Cas complex and the reporter are configured such that the Cas complex, the reporter, the cleaved detection moiety and the optional target are taken up by coacervates formed by the coacervate-forming compounds, or
B. the coacervate-forming compounds, the Cas complex and the reporter are configured such that the Cas complex, the reporter and the optional target are taken up by coacervates formed by the coacervate-forming compounds, or
C. the coacervate-forming compounds, the Cas complex and the reporter are configured such that the cleaved detection moiety, optionally the fluorophore, is taken up by coacervates formed by the coacervate-forming compounds, or
D. the coacervate-forming compounds, the Cas complex and the reporter are configured such that the Cas complex and the optional target are taken up by coacervates formed by the coacervate-forming compounds, or
E. the coacervate-forming compounds, the Cas complex and the reporter are configured such that the Cas complex is located at an interphase between the coacervate and the outside of the coacervate, or
F. the coacervate-forming compounds, the Cas complex and the reporter are configured such that the Cas complex and the optional target are taken up by coacervates formed by the coacervate-forming compounds.

11. The composition or kit of parts according to any of claims 1 to 10, wherein
(A)
a. the coacervate-forming compounds are positively charged,
b. the fluorophore of the reporter is negatively charged, and
c. the Cas protein is bound, optionally covalently bound, to a coacervate-forming
(B) compound; or
a. the coacervate-forming compounds are neutral in charge,
b. the Cas protein is not bound to a coacervate-forming compound, and
c. the fluorophore of the reporter is bound, optionally covalently bound, to a coacervate-forming compound; or
(C)
a. the coacervate-forming compounds are hydrophobic,
b. the Cas protein is not bound to a coacervate-forming compound, and
c. the reporter is bound, optionally covalently bound, to the fluorophore and comprises a hydrophobic molecule, optionally cholesterol, digoxigenin, biotin; or
(D)
a. the coacervate-forming compounds are positively charged,
b. the Cas protein is bound, optionally covalently bound, to a coacervate-forming compound or a negatively charged polymer; or
(E)
a. the coacervate-forming compounds are selected from positively charged compounds and from hydrophobic compounds,
b. the coacervate-forming compounds form two immiscible phases,
c. the Cas protein is bound, optionally covalently bound, to a coacervate-forming compound or a negatively charged polymer; or
d. the reporter is bound, optionally covalently bound, to the fluorophore and comprises a hydrophobic molecule.

12. A use of the composition or kit of parts according to any of claims 1 to 11 for
a. the detection and/or quantification of nucleic acids,
b. the detection of environmental DNA for monitoring and surveillance,
c. the detection of weeds, invasive species, plant viruses and/or plant virus infections,
d. the detection of DNA for personal identification,
e. the diagnosis of a disease, optionally an infection or a mutation,
f. the detection of antimicrobial resistances, and/or
g. the detection of environmental pollutants.

13. A method for the detection and/or quantification of nucleic acids comprising the steps:
(i) providing and mixing a composition according to any of claims 1 to 11 in an aqueous medium to form coacervates from the coacervate-forming compounds,
(ii) providing and mixing a nucleic acid target, optionally a cell lysate; a biofluid, optionally blood, saliva, urine, mucus, semen, vaginal fluid, feces, cerebrospinal fluid, amniotic fluid, bone marrow, milk, plasma, tears, sputum, serum, gastrointestinal fluid, or peritoneal fluid; Buccal, nasal, or vaginal swabs; wastewater, saltwater, freshwaters, soil, or soil extract; plant cells, hair, skin cells or microbiome cells with the composition of (i),
(iii) detecting and/or quantifying the nucleic acid target based on the signal of the detection moiety, optionally the fluorescent detection moiety.

14. The method according to claim 13, wherein the method is for the diagnosis of a disease, optionally an infection or a mutation, antimicrobial resistances, biological and/or environmental pollutants.
